# EUROPEAN PATENT SPECIFICATION

(11) **EP 1 002 071 B1**
(45) Date of publication and mention of the grant of the patent: **18.04.2007**
(21) Application number: 98942603.6
(22) Date of filing: 23.07.1998
(51) Int. Cl.: C12N 15/11, C12N 15/85, C12N 5/10, C12Q 1/68

(54) **HUMAN GLIAL CELL LINE-DERIVED NEUROTROPHIC FACTOR PROMOTERS, VECTORS CONTAINING SAME, AND METHODS OF SCREENING COMPOUNDS THEREWITH**
AUS EINER MENSCHLICHEN GLIAL ZELLLINIE-ABGELEITETER PROMOTOR EINES NEUROTROPHISCHEN FAKTORS, DIESEN ENTHALTENDE VEKTOREN UND METHODEN ZUM SCREENEN VON VERBINDUNGEN DAMIT
PROMOTEURS DE GNDF HUMAINS, VECTEURS CONTENANT CES PROMOTEURS ET PROCEDES DE SELECTION DE COMPOSES UTILISANT CES PROMOTEURS

(30) Priority: 05.08.1997 US 54812 P; 14.04.1998 US 81751 P
(43) Date of publication of application: 24.05.2000
(73) Proprietor: F. HOFFMANN-LA ROCHE AG, 4070 Basel (CH)
(72) Inventor: BAECKER, Preston, Albert, Los Altos, CA 94022 (US); JOHNSON, Randolph, Mellus, Half Moon Bay, CA 94019 (US); LEE, Walter, Hom, Campbell, CA 95008 (US); VERITY, Adrian, Neil, Sunnyvale, CA 94087 (US)
(74) Representative: Wächter, Dieter Ernst
(86) International application number: PCT/EP1998/004620
(87) International publication number: WO 1999/007843

(56) References cited:
- WO-A-93/06116
- WO-A-98/46737

### SEQUENCE LISTING

(1) GENERAL INFORMATION:
   (i) APPLICANT:
      (A) NAME: F. HOFFMANN-LA ROCHE AG
      (B) STREET: Grenzacherstrasse 124
      (C) CITY: Basle
      (D) STATE: BS
      (E) COUNTRY: Switzerland
      (F) POSTAL CODE (ZIP): CH-4010
      (G) TELEPHONE: 061-6884256
      (H) TELEFAX: 061-6881395
      (I) TELEX: 962292/965542 hlr ch
   (ii) TITLE OF INVENTION: HUMAN GLIAL CELL LINE-DERIVED NEUROTROPHIC FACTOR PROMOTER, VECTORS CONTAINING SAME, AND METHODS OF SCREENING COMPOUNDS THEREWITH
   (iii) NUMBER OF SEQUENCES: 30
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (v) CURRENT APPLICATION DATA:
      (A) APPLICATION NUMBER:
      (B) FILING DATE:
      (C) CLASSIFICATION:
(2) INFORMATION FOR SEQ ID NO:1:
   (i) SEQUENCE CHARACTERISTICS:
      (A) LENGTH: 1931 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
   GAATTCAGGT CCAATGGCTT CCGGAAAACA GGTTTCTGCT TAGCAAAGAC ATGCCCTATT 60
   TAGTACATTA TTTTAGAGGT ACAGCCAATT CCATGCCCCA TGTGAATGAA ATGTATTTAT 120
   GGTTATAGCC ATGCACAGGG TGTGTAAGGA CTTGCCCTCC TCCTGTCCTC TACAAAAGAA 180
   GGCTCAGGCA GCTTCTGGTG GTGAACTAAC CAACAAAAGG AATGCCCAGA AGGTCTCACC 240
   TCTCCCATCC ACAGAGCTCT GGAATGGGGG CCGGGCCCCT GATCGCTGGA AACTCAGCAT 300
   CCAAGTGGGC GCTTGCTGAA GTTTCCCATC TGCATTTTCG AAAATCTGGA TAAAAGCAGG 360
   TTTAGCTCAA CCTCCCCTAA CCCGTTCCTG ATAAAGTGAT CTTACGCCTC TGGAATTGGG 420
   ATTTTGAATG TATGTGCCTG CCCCATGTCT GGTATTAGAT TACAACAGCA TGAAAATGGA 480
   GCCTAGGACT GAGGAGCCGG CCAGCCTCAG AAGCCTTCTT CATTGCCTGC CATGTAATAC 540
   ATGATATGCA AAGCCTCTGA CTTCAGCCAG CAGATATTTG GAGACCGTGT TGGCCTTAGC 600
   ATGGGGACTA AACTAATGTG TGTTTAGGTC AGAAAGCGAA TCAGAAAAGC CACTGGAGGG 660
   CACGTCACGG AGTGAGAGCC CTATAAATTC ATCCCCACTC GCAGCGTCCC CTGGCAAATA 720
   CTCGGCTTCC CTCACCCGGC CGCTCCGCAC CTCTCCTTCT CGGTGCTAGA ACTCCGGCCG 780
   GCAGCTCCTT TTCTCGCCAC TGCCTCCTGC CCTGCGGAGA AGCGAGAGGG CGAGACACCC 840
   GAGCGGGAAG GCCGGCCTTC GACAGGCGCG AGGAAAGCCG GCGCTGCCTG CACCGGCAGA 900
   GCGCCACCCT CCCAGTGCGG CGCGCGGGGG CAAAGCCCGG CGCGGGCGCC GCGGTCAAGT 960
   TCGGATTGCC CCTTTCGGCT GGCCCGCGCA GAGCGCCGCA CATTCCTCTC GATTTTTGAT 1020
   CCACCCGGAG AGGAGAAGGC GCTAGCCCCG CGGCGTCTCC GCGCTCTCAG CGCTCTCCCT 1080
   GCAGCCCAGG GAGGGGCGCC TCGCGTCGCC CACTCGCACC CCGGCCCCCC GCCTCGGGAA 1140
   CTCCAGGGCT CCTCGCTGCT CTCCTCTCCT CCCCAACCGC CAGGGCCCAG CCGACGCCTC 1200
   CGACCCTCCC TCCCGGGGCC CGGCGCCTCC CCTCCCCCAC CCCGGACCCC GGACCCCCAG 1260
   CCCGGGCTCT AGAAGACCAG CCTGCGCTCC TGGCGCCCTC ATGTCTTCAC GGGACTCCCC 1320
   GCGCCGGTTG ACGTGGTGTC TCGTTCGGAT CTCCAGGCAA GACCTCAGCT CCGGCAGCAG 1380
   CATCAGACAA ACCAGTCTCG TGCTCCCAGG CAGTGCGCCC AGAGGAGGCG CAGAGCGCGG 1440
   CAGCTGCCGC TGAGCCGCCC GCAGCGCCCC GGGCCCGCGC AGCCCCAGCC AAGAGCGAGA 1500
   CGCGCGCAGC CTGTCAGCCC CCCACCCAAA GCAGCGGCGG CTGCTCGGAC TCGGCTTCTG 1560
   GGGGTGCGGG GGCCCGGCGG GAGAGTTGCC GGCAGCCCTC GCCCTGTTGG CGGCGGCGGC 1620
   GGCGGGATCT TGGCCGCCGC CTCCAGCGCG CCCTTGCTGC CCCGCGCGAC CCCAGGATTG 1680
   CGAACTCTTG CCCCTGACCT GTTGGGCGGG GCTCCGCGCT CCAGCCATCA GCCCGGATGG 1740
   GTCTCCTGGC TGGGACTTGG GGCACCTGGA GTTAATGTCC AACCTAGGGT CTGCGGAGAC 1800
   CCGATCCGAG GTAAGTGAGG GAGCGGGCCG GGGAGCAGGG AGCGGGTGGT GCCTGCGTCG 1860
   CGGGTGGGTG CGGCCGGCTG AAGGGCGCCC TAGTCAGAGG GTGCGTCTGA CCCCAGAGCA 1920
   AACGTGAGAG T 1931
(2) INFORMATION FOR SEQ ID NO:2:
   (i) SEQUENCE CHARACTERISTICS:
      (A) LENGTH: 2850 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 2717
      (D) OTHER INFORMATION: /note= "This position is N which indicates any nucleotide."
   (xi) SEQUENCE DESCRIPTION: SEQ ID NO:2:
   AAGCAGTTTG CACCGCGGGC AAAGGCCCAT GGTTGAGGAG AAGCGCGTCG GGCAGATGTA    60
   GATGTGTTTT GGGAAAGCGA TAGAGAGACA AACTGAGAAG CCGAGGGGTG GAGCCCACAC   120
   GGACTCTAAG AAAAACGTCC TAGTAGACGC TGTGTGTGCC GAGTCCCCCA ACCTCCCCCC   180
   TTTTCCCCCG CCCAGCAAAT ACCGAGAACG AAAGAGCCCC TCGGACTGGA GCCGGTCCCG   240
   GCGGCGCTCA GAGGGAAGAA TGCGCCCTCC CTGTCCCGGG GGAAGAGGGG AGCTCGATCC   300
   GGGCGCTGGC GGCGCTCGGC CGAGGGCGGG AACCGCAGCC GGGAGCCTCC TCTGGCGAGG   360
   CAGGGAGTTT TCCACTGTGT AGTCTTTTGC CAGCTTCGCC ACACTTCTAA TTGAGGATCT   420
   CCACGTCCAA TTTAAAAAGC CCTCCGTCGG CCAAGCAGGA AAAAAGGGAA AACCCTCTTA   480
   AGACGGAAGG GAAATCTAGT GCCTCTGTGG CTTCCTGCTG GCGGGCACCG CGATTTGGAA   540
   TGGCGCATTA ACCCCGCGCC TCCATGCTCC TCCGGTCGGA CCGCCAGCCA TTTCGCACGC   600
   ACCCGGGAGC TGGCACCGGC GGGGGGCTTG AGGGGGGAAG CTCGCGTTCC CCAGGTCCTA   660
   GCTGCCAAAG TACCTTTCTG GGCTCATTTT GCATGGCCTG GTGCAGTTTT CCTGTGTCTG   720
   CACATCGCGA CCCAGAACCT AGCTTTTTCC CGAGTTTGCA AACCAGCCCG CGAGGCAAGA   780
   GGCGCTCGGT GCTGCAGGTC ACTAGGAGTT TCTAGTCCCT ACACCCTCTC GAGCCCAACA   840
   GCTGCATAGC GAACAAACAG ACGCTCAGGA GATGTAAAAT GCATTAGTCT CTGCAGAGGT   900
   GGGAGGTGGC GGCCGAATTA AAGGCTTCCC CGGGTTGCCT GCACCGGGAC AGGGAGTGGG   960
   GTTCCGGGTA GAGATGAGGA CTGGAACCCT GGAATGGAGG CGGGGGTGCC TGTGAACTAA  1020
   TGGCTAGGAA AGGCACTGAG GTTTTTGCAT TAACCTGGGT TTTGCATTTC CCTCCCGCAT  1080
   GGGAAAAAAA AAATCGAGTT TTTCCTAAGA ACCGTTTTAT GGAGCCGGTC GAGGGGATAG    1140
   GATAATCGAC TGCACGAATC TCGCAGATTC CGCTTGAGGA GATTCTCTCT AGGTCACTAG    1200
   TGCCCTGGAG ACGAACCCTG GGATTAGGAA GGCACTGGAT AACACTGCAC CCCAAGATGC    1260
   CTCGGTTCTT CCCCGTCTCC TCATTGGTTG GAACTCGCCC CCAAATTTAC AGCCCCTTTT    1320
   TCTAACCCAC CCCCAGCTGC GTGGCCCTCC TTTGTAGGGG TGTGAGGATT GAGAAGCCAA    1380
   ACAGAGCCCA CCTCGGGCTG AAAAGAGCTG AACCCCCTAC TCTGCGCCGT ACCACGGTCT    1440
   AGGCCTTCCA GTGCCAGAGC ACCTCCAAAG CGTCCGAGAC TGGGTACAGT CGTCCAGGCG    1500
   TGACGGGGGC GCGGGGAGCC AGTGACTCCT CTGGGAGGGG AAGGGATTAG GGCCAGAATC    1560
   TCTCAAAGGT GCAAAAATCC AGTCAAGAGA GGGTTTTCGG GTATACCACG GAGGATTAAA    1620
   ACTTTCAAGA CAAATGCAGT CTTTGCCTAA CAGCAATGGT AAAGCATTTA CTGAGCTCTT    1680
   ACTACATGTT CCGCTAGAGC TTGTCATGCG TGATTTAATG TTCATAACTT TAAGAGGTGG    1740
   GAGGAGTATT TTAAATCGAA TCAGTGCTTT GGGAAGTTAA GTAACTTAAA CCAGATATCC    1800
   CATACGGGCC AAAAGTCTCC AAGTCCCTGC TAACTTCTTG CTCTCGCAAC AGAATACCTA    1860
   TTTAGGTGGG AAGAATGAGG TGTGGGCGGC AGGCTGGGTG AGTGGTGCCC CCGAGCCTGC    1920
   CCTCGACTAG CCAGAAGCCC GGTTGGGACC CGAGGCAGGG GAATGCGCTT GATTTTATTT    1980
   CCAAAGAGAA ACACCATCCT TGCTTGGGCC GAGGGCTCGT TCAGGGGCCT ATAGGAGCTA    2040
   CCGGGACAAG AAGGGGAGGT CTCTGGTTGG GGTGGAGGAC GAAGGGTGGG AACTACCCGA    2100
   TTGCCCCCCA GGAATGGGGG ATGTTGCGCA CCAGTAGAGG GGACTGGACA GGAATCGTGG    2160
   TGGTGGGGGT GGGGGGTTAA CTGGAGGGGA CAGCAGCCCT GCTTGAAACT CTGACCCCTA    2220
   AGACCGTGTG GTGGAGAAGG GCAGCTGCAA CCTGAACCAG GAGTGCGAGC TGCTCCTGGG    2280
   GCGCGCTGAG GAGGGAGAAG CGAACTGGGG ACTTGCAAGG AGGGCAGGAG TGCCCGAGGA    2340
   GCCGCTGGCC TGCAGCGGTG CCGGAGGAGG GCGGTGACGA GGTTGGAGAG GGGCGCAGGG    2400
   ACCCGCAGGG AGCCCAGGCT TAACGTGCAT TCTGCGGTTC TCTCCCCCAC CTCCCGCCTG    2460
   CCCGCGCAGG TGCCGCCGCC GGACGGGACT TTAAGATGAA GTTATGGGAT GTCGTGGCTG    2520
   TCTGCCTGGT GCTGCTCCAC ACCGCGTCCG CCTTCCCGCT GCCCGCCGGT AAGAGGCCTC    2580
   CCGAGGCGCC CGCCGAAGAC CGCTCCCTCG GCCGCCGCCG CGCGCCCTTC GCGCTGAGCA    2640
   GTGACTGTAA GAACCGTTCC CTCCCCGCGG GGGGGCCGCC GGCGGACCCC CTCGCACCCC    2700
   CACCCGCAGC CAGCCCNGCA CGTACCCCAA GCCAGCCTGA TGGCTGTGTG GCCTACCGAC    2760
   CCGTGGGCAA GGGGTGCGGG TGCTGAAGCC CCCAGGGGTG CCTGGCTGCC CACTGCTGCC 2820
   CGCACGCCTG GCCTGAAAGT GACACGCGCT 2850
(2) INFORMATION FOR SEQ ID NO:3:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
      GGTCTACGGA GACCGGATCC GAGGTGC 27
(2) INFORMATION FOR SEQ ID NO:4:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:4:
      TCTCTGGAGC CAGGGTCAGA TACATC 26
(2) INFORMATION FOR SEQ ID NO:5:
   (i) SEQUENCE CHARACTERISTICS:
      (A) LENGTH: 700 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
   GGTCTACGGA GACCGGATCC GAGGTGCCGC CGCCGGACGG GACTTTAAGA TGAAGTTATG 60
   GGATGTCGTG GCTGTCTGCC TGGTGCTGCT CCACACCGCG TCCGCCTTCC CGCTGCCCGC 120
   CGGTAAGAGG CCTCCCGAGG CGCCCGCCGA AGACCGCTCC CTCGGCCGCC GCCGCGCGCC 180
   CTTCGCGCTG AGCAGTGACT CAAATATGCC AGAGGATTAT CCTGATCAGT TCGATGATGT 240
   CATGGATTTT ATTCAAGCCA CCATTAAAAG ACTGAAAAGG TCACCAGATA AACAAATGGC 300
   AGTGCTTCCT AGAAGAGAGC GGAATCGGCA GGCTGCAGCT GCCAACCCAG AGAATTCCAG 360
   AGGAAAAGGT CGGAGAGGCC AGAGGGGCAA AAACCGGGGT TGTGTCTTAA CTGCAATACA 420
   TTTAAATGTC ACTGACTTGG GTCTGGGCTA TGAAACCAAG GAGGAACTGA TTTTTAGGTA 480
   CTGCAGCGGC TCTTGCGATG CAGCTGAGAC AACGTACGAC AAAATATTGA AAAACTTATC 540
   CAGAAATAGA AGGCTGGTGA GTGACAAAGT AGGGCAGGCA TGTTGCAGAC CCATCGCCTT 600
   TGATGATGAC CTGTCGTTTT TAGATGATAA CCTGGTTTAC CATATTCTAA GAAAGCATTC 660
   CGCTAAAAGG TGTGGATGTA TCTGACCCTG GCTCCAGAGA 700
(2) INFORMATION FOR SEQ ID NO:6:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:6:
      CAGCACCAGG CAGACAGC 18
(2) INFORMATION FOR SEQ ID NO:7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
      GCCACGACAT CCCATAACTT 20
(2) INFORMATION FOR SEQ ID NO:8:
   (i) SEQUENCE CHARACTERISTICS:
      (A) LENGTH: 721 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:8:
   ATCCCATACA GGCCAAAAGT CTCCAAGTCC CTGCTAACTT CTTGCTCTCG CAACAGAATA     60
   CCTATTTAGG CGGGAAGAAT GAGGTGTGGG CGGCAGGCTG GGTGAGTGGT GCCCCCGAGC    120
   CTGCCCTCGA CTAGCCAGAA GCCCGGTTGG GACCCGAGGC AGGGGAATGC GCTTGATTTT    180
   ATTTCCAAAG AGAAACACCA TCCTTGCTTG GGCCGAGGGC TCGTTCAGGG GCCTATAGGA    240
   GCTACCGGGA CAAGAAGGGG AGGTCTCTGG TTGGGGTGGA GGACGAAGGG TGGGAACTAC    300
   CCGATTGCCC CCCAGGAATG GGGATGTTGC GCACCAGTAG AGGGGACTGG ACAGGAATCG    360
   TGGTGGTGGG GGTGGGGGGT TAACTGGAGG GGACAGCAGC CCTGCTTGAA ACTCTGACCC    420
   CTAAGACCGT GTGGTGGAGA AGGGCAGCTG CAACCTGAAC CAGGAGTGCG AGCTGCTCCT    480
   GGGGCGCGCT GAGGAGGGAG AAGCGAACTG GGGACTTGCA AGGAGGGCAG GAGTGCCCGA    540
   GGAGCCGCTG GCCTGCAGCG GTGCCGGAGG AGGGCGGTGA CGAGGTTGGA GAGGGGCGCA    600
   GGGACCCGCA GGGAGCCCAG GCTTAACGTG CATTCTGCGG TTCTCTCCCC CACCTCCCGC    660
   CTGCCCGCGC AGGTGCCGCC GCCGGACGGG ACTTTAAGAT GAAGTTATGG GATGTCGTGG    720
   C                                                                    721
(2) INFORMATION FOR SEQ ID NO:9:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:9:
      YYYYYYYNYA GG 12
(2) INFORMATION FOR SEQ ID NO:10:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:10:
      TATGCCAGAG GATTATCCTG ATCAG 25
(2) INFORMATION FOR SEQ ID NO:11:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
      TAGCCCAGAC CCAAGTCAGT GAC 23
(2) INFORMATION FOR SEQ ID NO:12:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:12:
      CCCTGCTTGA AACTCTGACC 20
(2) INFORMATION FOR SEQ ID NO:13:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc_feature
      (B) LOCATION: 8
      (D) OTHER INFORMATION: /note= "This position is I which is inosine."
   (xi) SEQUENCE DESCRIPTION: SEQ ID NO:13:
      GGTCTACNGA GACCGGATCC GAGGT 25
(2) INFORMATION FOR SEQ ID NO:14:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:14:
      CGGCCCAAGC AAGGACGGTG TTTCT 25
(2) INFORMATION FOR SEQ ID NO:15:
   (i) SEQUENCE CHARACTERISTICS:
      (A) LENGTH: 776 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:15:
   CCAGCCTGCG CTCCTGGCGC CCTCATGTCT TCACGGGACT CCCCGCGCCG GTTGACGTGG   60
   TGTCTCGTTC GGATCTCCAG GCAAGACCTC AGCTCCGGCA GCAGCATCAG ACAAACCAGT  120
   CTCGTGCTCC CAGGCAGTGC GCCCAGAGGA GGCGCAGAGC GCGGCAGCTG CCGCTGAGCC  180
   GCCCGCAGCG CCCCGGGCCC GCGCAGCCCC AGCCAAGAGC GAGACGCGCG CAGCCTGTCA  240
   GCCCCCCACC CAAAGCAGCG GCGGCTGCTC GGACTCGGCT TCTGGGGGTG CGGGGGCCCG  300
   GCGGGAGAGT TGCCGGCAGC CCTCGCCCTG TTGGCGGCGG CGGCGGCGGG ATCTTGGCCG  360
   CCGCCTCCAG CGCGCCCTTG CTGCCCCGCG CGACCCCAGG ATTGCGAACT CTTGCCCCTG  420
   ACCTGTTGGG CGGGGCTCCG CGCTCCAGCC ATCAGCCCGG ATGGGTCTCC TGGCTGGGAC  480
   TTGGGGCACC TGGAGTTAAT GTCCAACCTA GGGTCTGCGG AGACCCGATC CGAGGTAAGT  540
   GAGGGAGCGG GCCGGGGAGC AGGGAGCGGG TGGTGCCTGC GTCGCGGGTG GGTGCGGCCG  600
   GCTGAAGGGC GCCCTAGTCA GAGGGTGCGT CTGACCCCAG AGCAAACGTG AGAGTAGAAA  660
   GTGGAAAGAC TAGAGGGAAC CTAAGCTATG CGAGGAGAGA GAAAAGTAGA ACCGGAGAGA  720
   GGCCGGCCTT TCGGAGGGGC ATGTTGCCTT TTCCACGTGG GCAAAGATCT GTGGAG      776
(2) INFORMATION FOR SEQ ID NO:16:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:16:
      GGTCATCATC AAAGGCGATG GGT 23
(2) INFORMATION FOR SEQ ID NO:17:
   (i) SEQUENCE CHARACTERISTICS:
      (A) LENGTH: 762 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:17:
   CCGCCTCCAG CGCGCCCTTG CTGCCCCGCG CGACCCCAGG ATTGCGAACT CTTGCCCCTG    60
   ACCTGTTGGG CGGGGCTCCG CGCTCCAGCC ATCAGCCCGG ATGGGTCTCC TGGCTGGGAC   120
   TTGGGGCACC TGGAGTTAAT GTCCAACCTA GGGTCTGCGG AGACCCGATC CGAGGTGCCG   180
   CCGCCGGACG GGACTTTAAG ATGAAGTTAT GGGATGTCGT GGCTGTCTGC CTGGTGCTGC   240
   TCCACACCGC GTCCGCCTTC CCGCTGCCCG CCGGTAAGAG GCCTCCCGAG GCGCCCGCCG   300
   AAGACCGCTC CCTCGGCCGC CGCCGCGCGC CCTTCGCGCT GAGCAGTGAC TCAAATATGC   360
   CAGAGGATTA TCCTGATCAG TTCGATGATG TCATGGATTT TATTCAAGCC ACCATTAAAA   420
   GACTGAAAAG GTCACCAGAT AAACAAATGG CAGTGCTTCC TAGAAGAGAG CGGAATCGGC   480
   AGGCTGCAGC TGCCAACCCA GAGAATTCCA GAGGAAAAGG TCGGAGAGGC CAGAGGGGCA   540
   AAAACCGGGG TTGTGTCTTA ACTGCAATAC ATTTAAATGT CACTGACTTG GGTCTGGGCT   600
   ATGAAACCAA GGAGGAACTG ATTTTTAGGT ACTGCAGCGG CTCTTGCGAT GCAGCTGAGA   660
   CAACGTACGA CAAAATATTG AAAAACTTAT CCAGAAATAG AAGGCTGGTG AGTGACAAAG   720
   TAGGGCAGGC ATGTTGCAGA CCCATCGCCT TTGATGATGA CC                      762
(2) INFORMATION FOR SEQ ID NO:18:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:18:
      GGTCATCATC AAAGGCGATG GGT 23
(2) INFORMATION FOR SEQ ID NO:19:
   (i) SEQUENCE CHARACTERISTICS:
      (A) LENGTH: 607 base pairs
      (B) TYPE: nucleic acid
      (C) STRANDECNESS: single
      (D) TOPOLOGY: linear
   (ii) MOLECULE TYPE: DNA (genomic)
   (xi) SEQUENCE DESCRIPTION: SEQ ID NO:19:
   CATACAGGCC AAAAGTCTCC AAGTCCCTGC TAACTTCTTG CTCTCGCAAC AGAATACCTA    60
   TTTAGGTGGG AAGAATGAGG TGTGGGCGGC AGGCTGGGTG CCGCCGCCGG ACGGGACTTT   120
   AAGATGAAGT TATGGGATGT CGTGGCTGTC TGCCTGGTGC TGCTCCACAC CGCGTCCGCC   180
   TTCCCGCTGC CCGCCGCAAA TATGCCAGAG GATTATCCTG ATCAGTTCGA TGATGTCATG   240
   GATTTTATTC AAGCCACCAT TAAAAGACTG AAAAGGTCAC CAGATAAACA AATGGCAGTG   300
   CTTCCTAGAA GAGAGCGGAA TCGGCAGGCT GCAGCTGCCA ACCCAGAGAA TTCCAGAGGA   360
   AAAGGTCGGA GAGGCCAGAG GGGCAAAAAC CGGGGTTGTG TCTTAACTGC AATACATTTA   420
   AATGTCACTG ACTTGGGTCT GGGCTATGAA ACCAAGGAGG AACTGATTTT TAGGTACTGC   480
   AGCGGCTCTT GCGATGCAGC TGAGACAACG TACGACAAAA TATTGAAAAA CTTATCCAGA   540
   AATAGAAGGC TGGTGAGTGA CAAAGTAGGG CAGGCATGTT GCAGACCCAT CGCCTTTGAT   600
   GATGACC                                                             607
(2) INFORMATION FOR SEQ ID NO:20:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:20:
      CCTCTGAAGG TTCCAGAATC GATAG 25
(2) INFORMATION FOR SEQ ID NO:21:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:21:
      GGAACCTCGA GAATCGATAG TGAATTCGTG 30
(2) INFORMATION FOR SEQ ID NO:22:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:22:
      TCGTACGTTG TCTCAGCTGC ATC 23
(2) INFORMATION FOR SEQ ID NO:23:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:23:
      ACCTTTTCCT CTGGAATTCT CTG 23
(2) INFORMATION FOR SEQ ID NO:24:
   (i) SEQUENCE CHARACTERISTICS:
      (A) LENGTH: 535 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:24:
   GGAACCTCGA GAATCGATAG TGAATTCGTG GGGGAGAGGG GCGCAGGGAC CCGCAGGGAG     60
   CCCAGGCTTA ACGTGCATTC TGCGGTTCTC TCCCCCACCT CCCGCCTGCC CGCGCAGGTG    120
   CCGCCGCCGG ACGGGACTTT AAGATGAAGT TATGGGATGT CGTGGCTGTC TGCCTGGTGC    180
   TGCTCCACAC CGCGTCCGCC TTCCCGCTGC CCGCCGCAAA TATGCCAGAG GATTATCCTG    240
   ATCAGTTCGA TGATGTCATG GATTTTATTC AAGCCACCAT TAAAAGACTG AAAAGGTCAC    300
   CAGATAAACA AATGGCAGTG CTTCCTAGAA GAGAGCGGAA TCGGCAGGCT GCAGCTGCCA 360
   ACCCAGAGAA TTCCAGAGGA AAAGGTCGGA GAGGCCAGAG GGGCAAAAAC CGGGGTTGTG 420
   TCTTAACTGC AATACATTTA AATGTCACTG ACTTGGGTCT GGGCTATGAA ACCAAGGAGG 480
   AACTGATTTT TAGGTACTGC AGCGGCTCTT GCGATGCAGC TGAGACAACG TACGA 535
(2) INFORMATION FOR SEQ ID NO:25:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:25:
      CCAGATATCC CATACAGGCC AA 22
(2) INFORMATION FOR SEQ ID NO:26:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:26:
      ATAACTAGAT CTTAAAGTCC CGTCC 25
(2) INFORMATION FOR SEQ ID NO:27:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:27:
      CCCAGGATTG CGAACTCTTG C 21
(2) INFORMATION FOR SEQ ID NO:28:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:28:
      TCTCCAAGTC CCTGCTAACT TCT 23
(2) INFORMATION FOR SEQ ID NO:29:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:29:
      CGTGCATTCT GCGGTTCT 18
(2) INFORMATION FOR SEQ ID NO:30:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:30:
      GAGCAGCACC AGGCAGAC 18

## Description

The present invention relates generally to neurotrophic factors. More particularly, the invention relates to human glial cell line-derived neurotrophic factor (GDNF) promoters, to reporter gene constructs containing a human GDNF promoter and to methods of using the construct to screen compounds for therapeutic efficacy in treating Parkinson's disease or other central and peripheral neurodegenerative diseases.

Parkinson's disease is a progressive neurodegenerative disease of unknown etiology affecting an estimated 600,000 to 1,000,000 in the United States. The disease affects both men and women with no apparent class or racial preferences. Parkinson's disease is characterized by symptoms such as muscle tremors, muscle weakness, rigidity, slowness of movement (bradykinesia) with alterations in posture and equilibrium. Without treatment, afflicted individuals progressively deteriorate over 5-10 years to a rigid, akinetic state requiring constant care. Death frequently results from complications resulting from lack of mobility. At present there is no diagnostic test for Parkinson's disease and all treatments are palliative rather than curative. Pathophysiologically, Parkinson's disease presents as a severe loss of the pigmented, dopaminergic neurons within the substantia nigra pars compacta and the melanin-containing neurons of the locus coeruleus and the dorsal motor nucleus of the vagus. The principal neuropathologic lesion in Parkinson's disease is loss of dopaminergic neurons resulting in depletion of dopaminergic terminals in the striatum. This selective neuronal loss results in reduced levels of the neurotransmitter dopamine within the striatum.

Presently, the most effective treatment for alleviating the symptoms of Parkinson's disease is the oral administration of L-3,4-dihydroxyphenylalanine (L-DOPA) in combination with a peripherally acting inhibitor of aromatic L-amino acid decarboxylase. L-DOPA, unlike dopamine, is actively transported across the blood brain barrier and into presynaptic terminals of dopaminergic neurons, within which L-DOPA is decarboxylated to dopamine and sequestered into storage vesicles by a transporter protein. L-DOPA treatment diminishes all symptoms of Parkinson's disease particularly if treatment is initiated early in the course of the disease.

Glial cell line-derived neurotrophic factor (GDNF), a distant member of the transforming growth factor-⁻ (TGF-⁻) superfamily, was originally isolated by virtue of its ability to induce dopamine uptake and cell survival in cultures of embryonic ventral midbrain dopaminergic neurons. GDNF has been purified and sequenced from conditioned media from rat B49 glial cells (Lin et al. (1993), *Science* 260, 1130), and the rat GDNF cDNA has been cloned and used to isolate the protein encoding portions of the gene from a human genomic library (International Publication No. WO 93/06116; GenBank Accession Nos. L19062 and L19063).

GDNF is an approximately 39 kD glycosylated protein that exists as a homodimer in its native form. In humans and rodents a single gene gives rise to alternatively spliced forms. Both forms contain a consensus signal peptide sequence and a consensus sequence for proteolytic processing. Proteolytic cleavage yields identical mature 134 amino acid residue forms.

GDNF has a significant role in the development and differentiation of the mammalian nervous system. GDNF promotes dopamine uptake and cell survival of embryonic mesencephalic dopaminergic neurons as well as the survival and/or differentiation of a wide range of central and peripheral neuronal and nonneuronal cell populations. Within the central nervous system, *in vitro* studies support a developmental role for GDNF in the survival of mid-brain dopaminergic neurons, cerebellar Purkinje neurons, and cranial and spinal cord motor neurons. In the peripheral nervous system, GDNF supports the development of multiple neuronal populations including sympathetic, parasympathetic, sensory and autonomic neurons.

Localization of GDNF mRNA transcripts to GDNF-sensitive neuronal target fields is consistent with an *in vivo* function of GDNF as a target-derived neurotrophic factor. In addition, studies using GDNF-null mice have confirmed the pleiotropic roles of GDNF in peripheral neuronal development as well as its vital role in kidney ontogeny during embryonic development.

Although the importance of GDNF during development has been well defined, the function of GDNF in the adult is less clear. Of clinical importance, exogenous GDNF protects mesencephalic dopaminergic neurons against axotomy-induced degeneration in the adult rat brain and 1-methyl-4-phenyl-1,2,3,6-tetrahydropyridine (MPTP)-induced degeneration of nigral dopaminergic neurons in rodents. In addition, GDNF injected intracerebrally into rhesus monkeys that exhibit MPTP-induced Parkinson's disease-like symptoms produces significant improvements in bradykinesia, rigidity and postural instability. Based at least in part on these results, delivery of recombinant GDNF has been in clinical trials for the treatment of Parkinson's disease.

Little is known about the cellular and molecular events that regulate GDNF expression. At the cellular level, GDNF mRNA has been localized to both neuronal and astrocytic cells and, like many neurotrophic factors, expression is elevated in injured tissues. In response to excitatory amino acids, GDNF mRNA levels are elevated in hippocampal neurons and hippocampal and striatal primary astrocytes.

Due to its relatively large size, GDNF does not cross the blood-brain barrier, making direct administration into the brain the only viable means of delivery. This necessitates stereotaxic injection of GDNF into the brain or intracerebroventricular (ICV) delivery of GDNF which severely limits its therapeutic utility. On the other hand, a noninvasive therapy to protect substantia nigra neurons from cell death, for example, small molecules aimed at specifically increasing endogenous GDNF levels and/or its release, would be preferred. Ideally, such a small molecule can be administered by any convenient route, cross the blood brain barrier and increase the local synthesis of GDNF, thereby sparing the target population of dopaminergic neurons at risk in Parkinson's disease. Such therapy would be less pulsatile and less invasive than intraparenchymal, ICV, or intrathecal injection of GDNF.

There is a need in the art for an alternative means by which tissue levels of GDNF may be increased. One such method is to enhance endogenous GDNF levels by inducing GDNF gene expression at the transcriptional level.

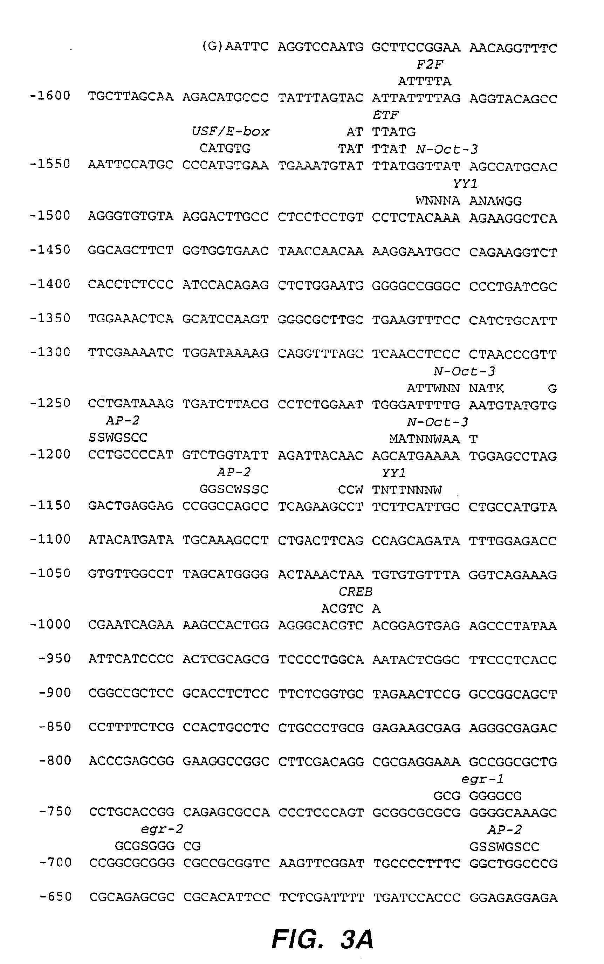

WO 98/46737 describes the cDNA of human GDNF gene and a promoter sequence. The disclosed promoter sequence is incomplete, missing e.g. the TATAA box.
Accordingly, for the first time it has been identified, sequenced, characterized and cloned a proximal promoter and a distal promoter from the human GDNF gene. The distal and proximal promoters contain sequences characteristic of eukaryotic gene regulatory elements. For example, the promoters include transcription factor binding site consensus sequences. The distal promoter comprises the promoter nucleotide sequence set forth at positions -62 through -1 of Figures 3A-3B (SEQ ID NO:1), the promoter nucleotide sequence set forth at positions -363 through -1 of Figures 3A-3B (SEQ ID NO:1), or the promoter nucleotide sequence set forth at positions -1635 through -1 of Figures 3A-3B (SEQ ID NO:1).

In addition, it has been prepared a recombinant construct in which the promoter is operably linked to a reporter gene that encodes a polypeptide detectable by any of a variety of techniques well known in the art. The construct has been transfected into human neuronal and glial cells by which candidate compounds may be screened for their ability to stimulate expression of a gene under control of the promoter. In this manner, the invention provides a means and a method by which compounds capable of inducing GDNF gene expression *in vivo* may be identified. Such compounds are useful in treating central and peripheral neurodegenerative diseases as well as renal, urogenital and gastrointestinal diseases, and neurodegenerative sequelae of physical nerve trauma, when administered to a mammalian subject in an acceptable pharmaceutical formulation.

Furthermore, the invention provides a method for identifying a compound that modulates the expression of GDNF. The method involves (a) introducing into a host cell a DNA fragment comprising a human distal GDNF promoter operably linked to a reporter gene; (b) mixing a test compound with the host cell and culturing the host cell under conditions whereby the reporter gene is capable of expression; and (c) comparing the level of expressed reporter gene product in the presence and absence of the test compound; wherein a change in the level of expression of the reporter gene product indicates that the test compound is capable of modulating the level of GDNF expression.

These and other embodiments of the present invention will readily occur to those of ordinary skill in the art in view of the disclosure herein.

### Brief Description of the Figures

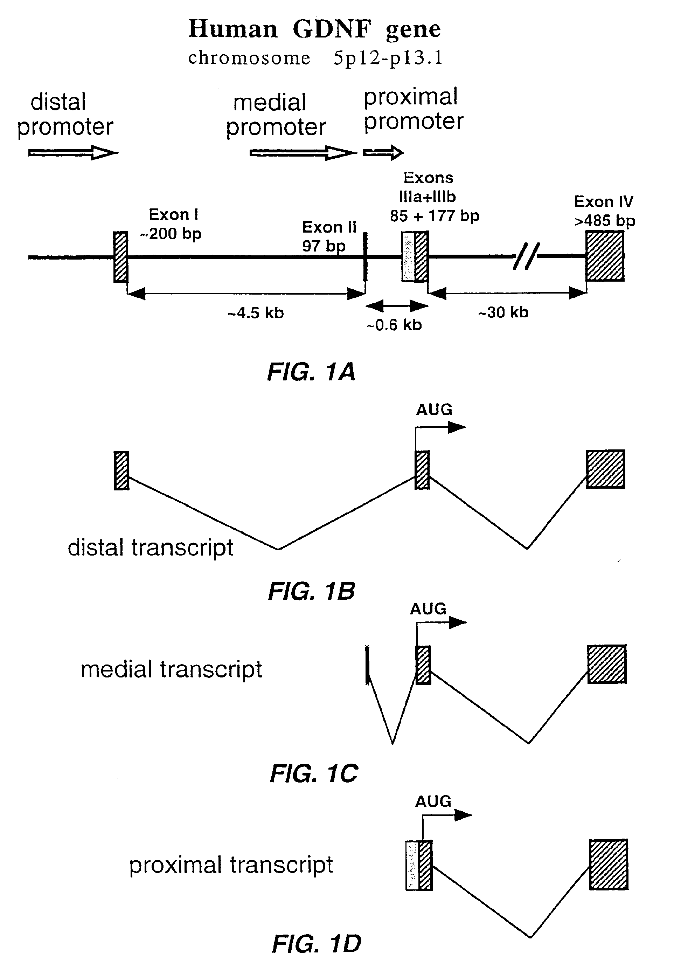

Figures 1A-1D depict the structure of the various human GDNF promoters. Figure 1A depicts a map of the human GDNF gene showing the location of exons I, II, IIIa+IIIb and IV, and the distal, medial and proximal promoters. Figures 1B, 1C and 1D are schematic diagrams illustrating GDNF gene transcripts that originate from the distal GDNF promoter, the medial GDNF promoter and the proximal GDNF promoter, respectively.

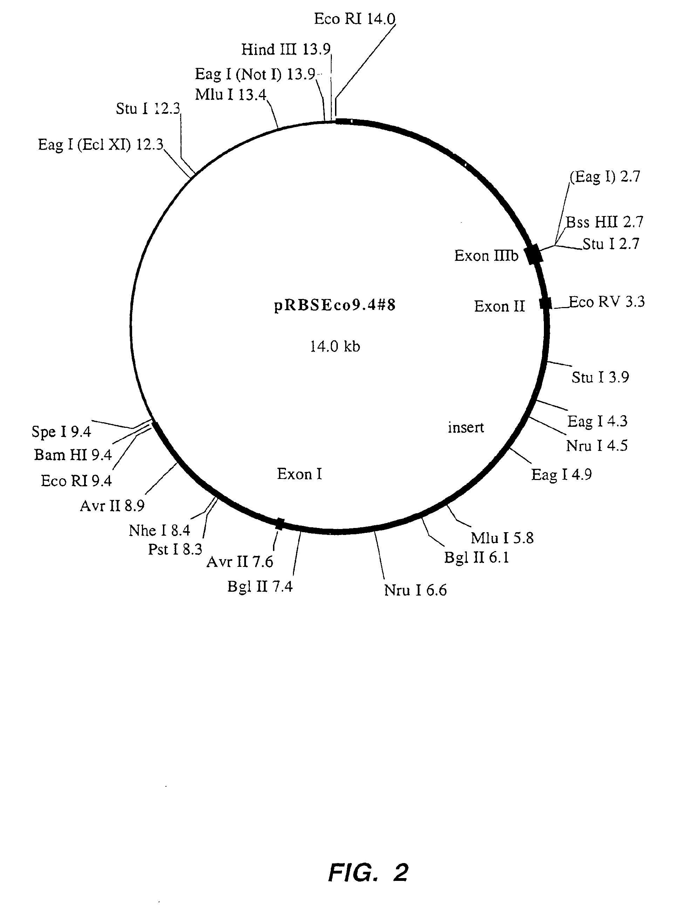

Figure 2 is a diagram of plasmid pRBS*Eco*9.4#8.
Figures 3A-3B (SEQ ID NO:1) show the nucleotide sequence of the human distal GDNF promoter, and a portion of the first intron. Exon I (starting at nt Consensus sequences for transcription factors are given above the DNA sequence and the abbreviated name of the transcription factor is shown in italics above the consensus sequence. Nucleotide numbering is relative to the start of exon 1.

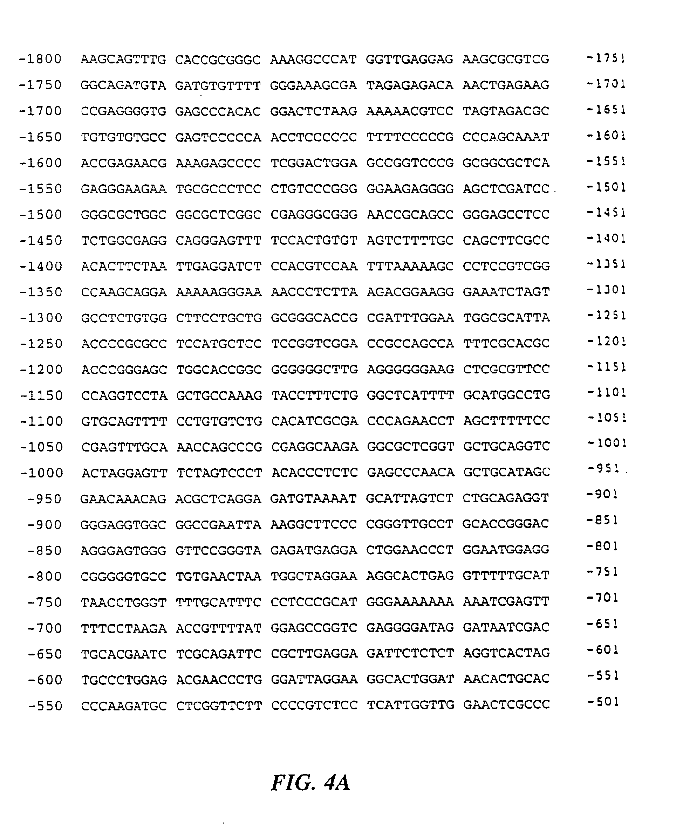

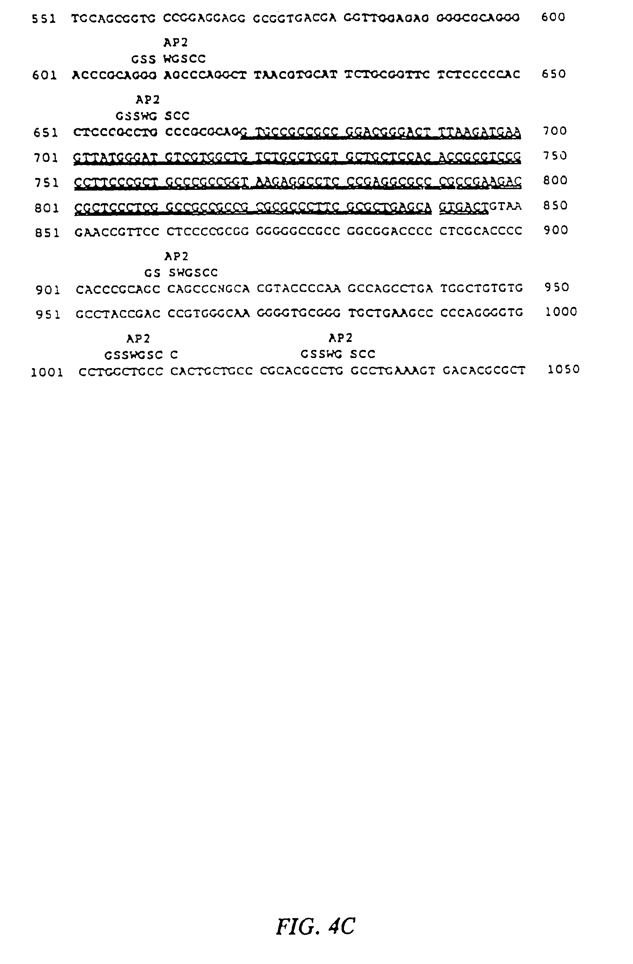

Figures 4A-4C (SEQ ID NO:2), show the nucleotide sequence of the human proximal GDNF promoter in which exon II is underlined (the medial transcript), exon III is double underlined, the beginning of the proximal transcript (originating at exon IIIa) is indicated in bold, and the names of putative transcription factors are given above their consensus binding sites. Numbering is relative to the start of exon II.

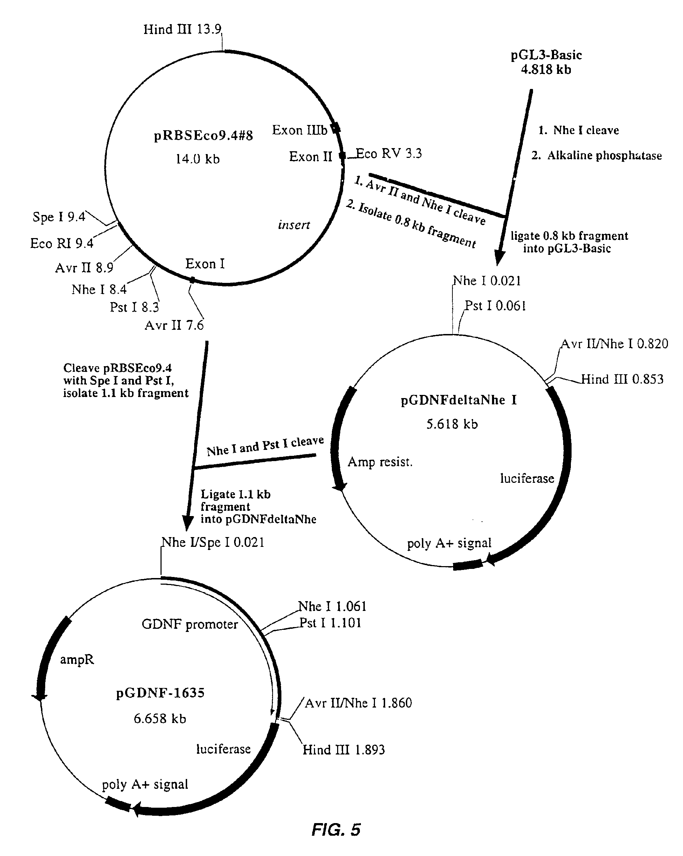

Figure 5 shows the strategy for making plasmid pGDNF-1635 as described in Example 2.

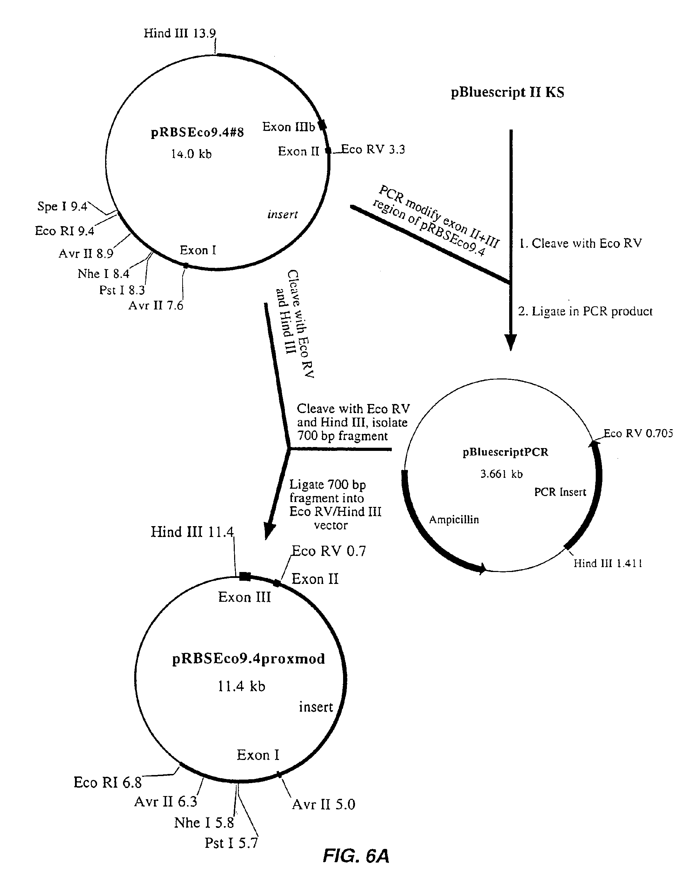

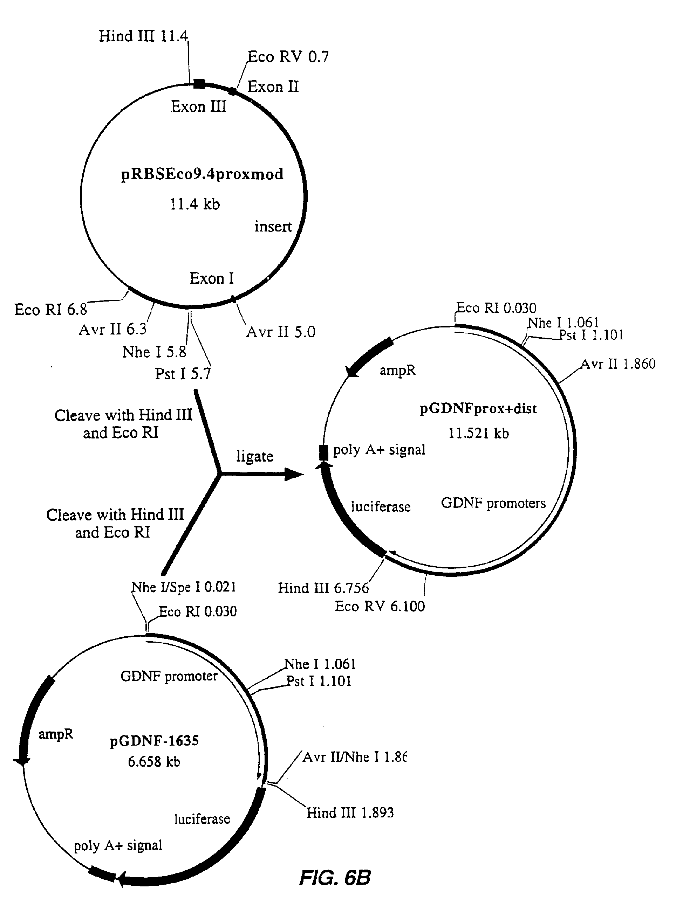

Figures 6A-6B show the strategy for making plasmid pGDNFprox+dist as described in Example 5.

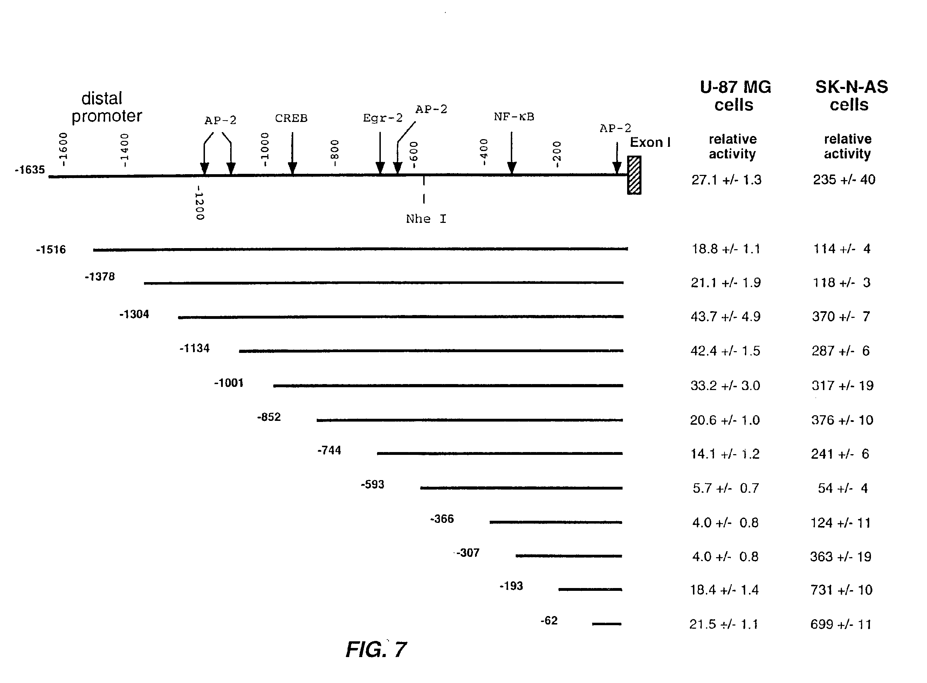

Figure 7 shows a map of the human GDNF distal promoter and various 5' deletions thereof. The top line is a linear depiction of the full 5'-flanking fragment with locations of some possible binding sites for transcription factors indicated. Each succeeding line shows a linear map of the amount of 5'-flanking sequence left in a deletion, with an indication of the extent of the most 5'-sequence (as shown in Figures 3A-3B), given at the left of each subclone. All clones were in the luciferase reporter vector, pGL3, as described in the examples. Relative luciferase mean values +/- sem (n=6), in two different hosts, are given for each deletion at the right.

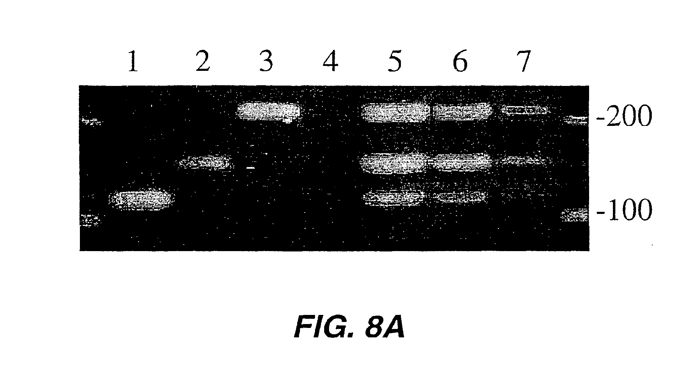

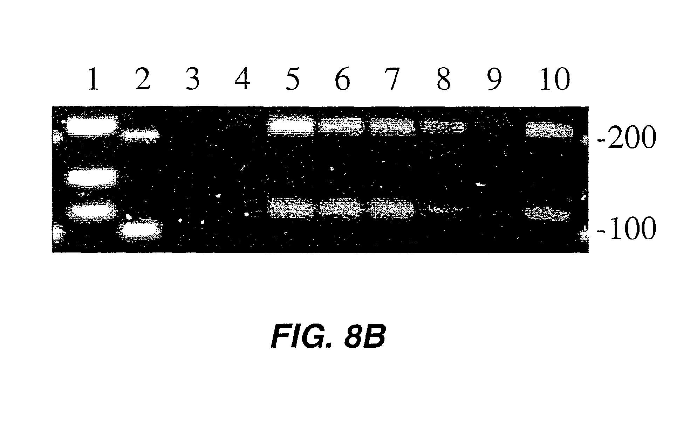

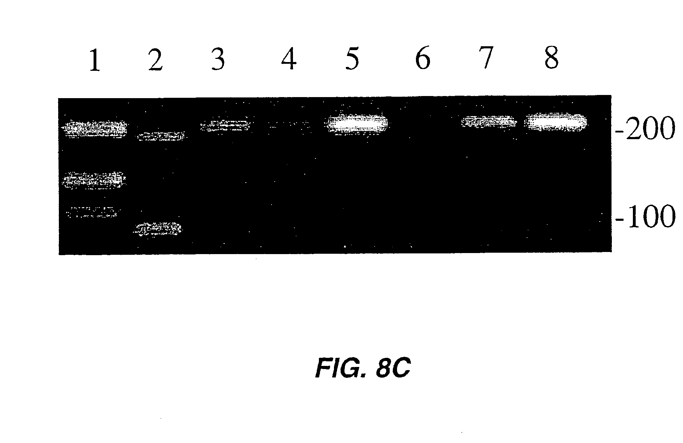

Figures 8A-8C depict RT-PCR results for three different GDNF transcripts, as described in Example 7. Figure 8A shows calibration results with cloned cDNA templates: Lane 1, 100 zeptomoles proximal template plus all primers; lane 2, 100 zeptomoles medial template plus all primers; lane 3, 100 zeptomoles distal template plus all primers; lane 4, no template; lane 5, 100 zeptomoles each template plus all primers; lane 6, 10 zeptomoles each template plus all primers; lane 7, 1 zeptomole each template plus all primers. Figure 8B shows U-87 MG cell results after 12 hours of various treatments: Lane 1, positive control of 10 zeptomoles each cloned template plus all primers; lane 2, 100 and 200 hp markers; lane 3, negative control as in lane 4, but without reverse transcriptase treatment; lane 4, media alone; lane 5, IL-1⁻ (10 ng/ml); lane 6, PDD (10 nM); lane 7, BFGF (50 ng/ml); lane 8, db-cAMP (1 mM); lane 9, dexamethasone (1 M); lane 10, TNF^ (10 ng/ml). Figure 8C shows results from human tissues: Lane 1, positive control of 10 zeptomoles each cloned template; lane 2, 100 and 200 hp markers; lane 3, fetal liver; lane 4, adult liver; lane, 5, fetal kidney; lane 6, adult brain; lane 7, fetal brain; and lane 8, adult skeletal muscle.

The practice of the present invention will employ, unless otherwise indicated, conventional techniques of protein chemistry and biochemistry, molecular biology, microbiology and recombinant DNA technology, which are within the skill of the art. Such techniques are explained fully in the literature. See, e.g., Sambrook et al., *Molecular Cloning: A Laboratory Manual,* Second Edition (1989); *DNA Cloning,* Vols. I and II (D.N. Glover ed. 1985); *Oligonucleotide Synthesis* (M.J. Gait ed. 1984); *Nucleic Acid Hybridization* (B.D. Hames & S.J. Higgins eds. 1984); *Animal Cell Culture* (R.K. Freshney ed. 1986); Schleif et al., *Practical Methods in Molecular Biology,* (Springer-Verlag, NY, 1981); *Immobilized Cells and Enzymes* (IRL press, 1986); *PCR: A Practical Approach* (McPherson et al. eds. (1991) IRL Press); Perbal, B., *A Practical Guide to Molecular Cloning* (1984); the series, *Methods In Enzymology* (S. Colowick and N. Kaplan eds., Academic Press, Inc.).

### I. Definitions and Nomenclature

Before the present invention is disclosed and described in detail, it is to be understood that this invention is not limited to specific assay formats, materials or reagents, as such may, of course, vary. It is also to be understood that the terminology used herein is for the purpose of describing particular embodiments only and is not intended to be limiting.

It must be noted that, as used in the specification and the appended claims, the singular forms "a," "an" and "the" include plural referents unless the context clearly dictates otherwise. Thus, for example, reference to a plasmid or vector containing "a GDNF promoter" includes plasmids or vectors containing more than one such promoter, reference to "a human cell line" includes more than one such cell line, reference to "a transcription initiation site" includes more than one transcription initiation site, and the like.

In this specification and in the claims which follow, reference will be made to a number of terms which shall be defined to have the following meanings:

A "GDNF promoter" is a DNA regulatory region capable of binding RNA polymerase and initiating transcription of a downstream (3'-direction) coding sequence. A GDNF promoter for purposes of the present invention is generally a DNA fragment of about 50 to about 200 bp, or greater, in the 5'-flanking DNA upstream of the transcriptional initiation start site. The promoter forms an initiation complex with RNA polymerase to initiate and drive transcription of the downstream sequence. The initiation complex can be modified by activating elements termed "enhancers" or inhibiting elements termed "suppressors." The term "promoter" includes activating and inhibiting elements, unless the context clearly dictates otherwise. For purposes of the present invention, the promoter sequence is bounded at its 3'-terminus by a transcription initiation site(s) (but does not necessarily include the initiation site which can be provided by a heterologous 5'-UTR) and extends upstream (5'-direction) to include fragments comprising a minimum number of bases or elements necessary to initiate transcription at levels detectable above background.

Furthermore, a "GDNF promoter" as used herein, intends sequences which include modifications, such as deletions, additions and substitutions, to the native sequence, so long as the promoter maintains the ability to initiate transcription at levels detectable above background. These modifications may be deliberate, as through site-directed mutagenesis, or may be accidental, such as through naturally occurring mutational events or errors due to PCR amplification.

For example, as described in the examples below and shown in the accompanying figures, consensus binding sites for a number of transcription factors have been found in the GDNF promoters described herein, including binding sites for epidermal growth factor receptor transcription factor (ETF), early growth response (egr) family members such as egr1 and egr2 (see, e.g., Liu et al. (1996), *Crit. Rev. Oncogen.* 7, 101), Sp1 (see, e.g., Dynan et al. (1983) *Cell* 35, 79; Briggs et al. (1986), *Science* 234, 47; Hagen et al., *EMBO J* 13, 3843), members of the CREB/ATF family (see, e.g., Papavassiliou (1994), *Anticancer Res.* 14, 1801), AP-2 (see, e.g., Mitchell et al. (1987), *Cell* 50, 847; Imagawa et al. (1987), *Cell* 51, 251; Williams et al. (1988), *Genes Dev.* 2, 1557) the nuclear factor κB (NF-κB) (see, e.g., Baeuerle et al. (1996), *Cell* 87, 13; Baldwin et al. (1996), *Ann. Rev. Immunol.* 14, 649), yin-yang-1 (YY-1) and GC factor (GCF). Several of these factors, such as ETF, GCF and YY-1, repress transcription. Thus, deletion or mutation of one or more regions including binding sites for these factors (specified in Figures 3A-3B and 4A-4C) may be desirable in order to enhance transcription of a gene operably linked to the promoter sequence.

For example, with reference to the distal promoter, as seen in Figure 7 and described in the examples, a promoter sequence truncated at position -62, numbered with reference to exon I (e.g., comprising the sequence at positions -62 through -1, of Figure 7), maintains high activity when used in human neuroblastoma SK-N-AS cells. Thus, a distal GDNF promoter for purposes of the present invention may comprise any of the deletions depicted in Figure 7, as well as intervening truncations, such as, e.g., promoter sequences truncated at position -63, -194, -308, -363, and so on, as long as the promoter retains the ability to initiate transcription at levels detectable above background in a given cell line. Specifically excluded from the definition of "GDNF promoter" as used herein are sequences having complete identity to the portion of the mouse GDNF sequence corresponding to the human GDNF sequence beginning at exon I and continuing upstream of exon I (see, e.g., GenBank entry #D88350S1).

A "distal" GDNF promoter is a GDNF promoter that is immediately adjacent to and upstream from the exon I of the human GDNF gene. A "medial" GDNF promoter is a GDNF promoter that is downstream from the distal GDNF promoter and immediately adjacent to and upstream from exon II of the human GDNF gene. A "proximal" GDNF promoter is a GDNF promoter that is downstream from exon II and upstream from exon IIIa+IIIb (alternately referred to herein as "exon III") of the human GDNF gene. As used herein, the phrase "proximal GDNF promoter" is intended to encompass both the medial GDNF promoter and the proximal GDNF promoter unless the meaning is clearly otherwise. Mapping of the human GDNF gene and promoters are described in greater detail below.

A "reporter gene" is a gene that, upon expression, confers a phenotype on a cell expressing the reporter gene, such that the cell can be identified under appropriate conditions. For example, the reporter gene may produce a polypeptide product that can be easily detected or measured in a routine assay. Suitable reporter genes known in the art which confer this characteristic include those that encode chloramphenicol acetyl transferase (CAT activity), galactosidase, luciferase, alkaline phosphatase, human growth hormone, fluorescent proteins, such as green fluorescent protein (GFP), and others. Indeed, any gene that encodes a protein or enzyme that can readily be measured, for example, by an immunoassay such as an enzyme-linked immunosorbent assay (ELISA) or by the enzymatic conversion of a substrate into a detectable product, and that is substantially not expressed in the host cells (specific expression with no background) can be used as a reporter gene to test for promoter activity. Other reporter genes for use herein include genes that allow selection of cells based on their ability to thrive in the presence or absence of a chemical or other agent that inhibits an essential cell function. Suitable markers, therefore, include genes coding for proteins which confer drug resistance or sensitivity thereto, or change the antigenic characteristics of those cells expressing the reporter gene when the cells are grown in an appropriate selective medium. For example, reporter genes include: cytotoxic and drug resistance markers, whereby cells are selected by their ability to grow on media containing one or more of the cytotoxins or drugs; auxotrophic markers by which cells are selected by their ability to grow on defined media with or without particular nutrients or supplements; and metabolic markers by which cells are selected for, e.g., their ability to grow on defined media containing the appropriate sugar as the sole carbon source. These and other reporter genes are well known in the art.

A "change in the level of reporter gene product" is shown by comparing expression levels of the reporter gene product in a cell exposed to a candidate compound relative to the levels of reporter gene product expressed in a cell that is not exposed to the test compound and/or to a cell that is exposed to a control compound. The change in level can be determined quantitatively for example, by measurement using a spectrophotometer, spectrofluorometer, luminometer, and the like, and will generally represent a statistically significant increase or decrease in the level from background. However, such a change may also be noted without quantitative measurement simply by, e.g., visualization, such as when the reporter gene is one that confers the ability on cells to form colored colonies on chromogenic substrates.

The term "expression" a.s used herein intends both transcriptional and translational processes, i.e., the production of messenger RNA and/or the production of protein therefrom.

"Parkinson's disease-like symptoms" include muscle tremors, muscle weakness, rigidity, bradykinesia, alterations in posture and equilibrium, dimentia and like symptoms generally associated with Parkinson's disease or other neurodegenerative diseases.

The term "polynucleotide" or "oligonucleotide" as used herein means a polymeric form of nucleotides of any length, either ribonucleotides or deoxyribonucleotides. This term refers only to the primary structure of the molecule. Thus, the term includes double- and single-stranded DNA, as well as double- and single-stranded RNA. It also includes modifications, such as by methylation and/or by capping, and unmodified forms of the polynucleotide.

"Recombinant host cells," "host cells," "cells," "cell lines," "cell cultures," and other such terms denoting microorganisms or higher eukaryotic cell lines cultured as unicellular entities refer to cells which can be, or have been, used as recipients for recombinant vectors or other transfer DNA, immaterial of the method by which the DNA is introduced into the cell or the subsequent disposition of the cell. The terms include the progeny of the original cell which has been transfected. Cells in primary culture as well as cells such as oocytes also can be used as recipients.

A "vector" is a replicon in which another polynucleotide segment is attached, such as to bring about the replication and/or expression of the attached segment. The term includes expression vectors, cloning vectors, and the like.

A "coding sequence" is a polynucleotide sequence that is transcribed into mRNA and translated into a polypeptide. The boundaries of the coding sequence are determined by a translation start codon at the 5'-terminus and a translation stop codon at the 3'-terminus. A coding sequence can include, but is not limited to, mRNA, cDNA, synthetic DNA, and recombinant polynucleotide sequences. Also included is genomic DNA where the coding sequence is interrupted by introns.

"Operably linked" refers to a situation wherein the components described are in a relationship permitting them to function in their intended manner. Thus, for example, a control sequence such as a promoter, "operably linked" to a coding sequence, is positioned in such a manner that expression of the coding sequence is achieved under conditions compatible with the control sequences. A coding sequence may be operably linked to control sequences that direct the transcription of the polynucleotide whereby said polynucleotide is expressed in a host cell. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence. An operably linked GDNF promoter will direct transcription of a nucleic acid molecule joined in proper reading frame thereto.

The term "transfection" refers to the insertion of an exogenous polynucleotide into a host cell, irrespective of the method used for the insertion, or the molecular form of the polynucleotide that is inserted. The insertion of a polynucleotide *per se* and the insertion of a plasmid or vector comprised of the exogenous polynucleotide are included. The exogenous polynucleotide may be directly transcribed and translated by the cell, maintained as a nonintegrated vector, for example, a plasmid, or alternatively, may be stably integrated into the host genome.

"Transfection" generally is used in reference to a eukaryotic cell while the term "transformation" is used to refer to the insertion of a polynucleotide into a prokaryotic cell.

The term "isolated," when referring to a polynucleotide or a polypeptide, intends that the indicated molecule is present in the substantial absence of other similar biological macromolecules. The term "isolated" as used herein means that at least 75 wt.%, more preferably at least 85 wt.%, more preferably still at least 95 wt.%, and most preferably at least 98 wt.% of a composition is the isolated polynucleotide or polypeptide. An "isolated polynucleotide" that encodes a particular polypeptide refers to a polynucleotide that is substantially free of other nucleic acid molecules that do not encode the subject polypeptide; however, the molecule may include functionally and/or structurally conservative mutations as defined herein.

Two polynucleotide sequences, or two fragments or segments of a polynucleotide sequence, are "substantially homologous" when at least about 65%, preferably at least about 75%, more preferably at least about 80 to 85%, and most preferably at least about 90% to 95% or more, of the nucleotides match over a defined length of the molecule. As used herein, substantially homologous also refers to sequences showing identity to the specified polynucleotide sequence. Polynucleotide sequences that are substantially homologous can be identified in a Southern hybridization experiment under, for example, stringent conditions, as defined for that particular system Defining appropriate hybridization conditions is within the skill of the art. See, e.g., Sambrook et al., supra; *DNA Cloning,* vols I & II, supra; *Nucleic Acid Hybridization,* supra.

Other techniques for determining nucleic acid sequence identity are well known in the art and include determining the nucleotide sequence of the polynucleotide of interest and comparing this to a second nucleotide sequence. Programs available in the Wisconsin Sequence Analysis Package, Version 8 (available from Genetics Computer Group, Madison, WI) for example, the BESTFIT, FASTA and GAP programs, are capable of calculating identity between two polynucleotides. Additionally, the database search tool, BLAST (Altschul, et al. (1990), *J. Mol. Biol.* 215, 403-410), may be employed to search gene data bases and determine percent identity between a given sequence and a sequence present in the database. Other programs for calculating identity or similarity between sequences are known in the art.

A sequence "functionally equivalent" to a GDNF promoter sequence is one which functions in the same manner as the corresponding GDNF promoter. Thus, a promoter sequence "functionally equivalent" to, e.g., a distal GDNF promoter described herein is one which is capable of directing transcription of a nucleic acid molecule joined in proper reading frame thereto above background levels.

### II. General Methods

Isolated DNA comprising a human distal GDNF promoter and proximal GDNF promoter, and a reporter gene construct comprising a GDNF promoter are provided herein. The invention also encompasses a method for screening compounds for GDNF promoter-inducing activity using the constructs, cells that have been transformed or transfected with the constructs.

As described in greater detail in Example 1, exon I of the human GDNF gene (see, Figure 1), and the distal promoter upstream thereof, can be identified, amplified, cloned and sequenced according to the following general scheme. The presence of GDNF mRNA in human tissues is demonstrated by reverse transcriptase-polymerase chain reaction ("RT-PCR") using oligonucleotide primers based on the 5'-end of rat GDNF cDNA. PCR-identification of a human GDNF gene sequence involves amplification of sequences from a human genomic or cDNA library. Degenerate or nondegenerate oligonucleotide primers for PCR may be prepared based on the sequence of rat GDNF, or any mammalian GDNF sequence that has been shown to be substantially homologous to a portion of the human GDNF. The products of such PCR reactions may be selected according to size by gel electrophoresis, cloned into an appropriate vector and the cloned DNA sequenced to identify the GDNF promoter sequence.

More particularly, PCR employs short oligonucleotide primers (generally 10-20 nucleotides in length) that match opposite ends of a desired sequence within a DNA molecule. The sequence between the

More particularly, PCR employs short oligonucleotide primers (generally 10-20 nucleotides in length) that match opposite ends of a desired sequence within a DNA molecule. The sequence between the primers need not be known. The initial template can be either RNA or DNA. If RNA is used, it is first reverse transcribed to cDNA. The cDNA is then denatured, using well-known techniques such as heat, and appropriate oligonucleotide primers are added in molar excess.

Primers hybridize to the complementary target polynucleotide and primer extension is effected using DNA polymerase in the presence of deoxynucleotide triphosphates or nucleotide analogs. The resulting product includes the respective primers at their 5'-termini, covalently linked to the newly synthesized complements of the original strands. The replicated molecule is again denatured, hybridized with primers, and so on, until the product is sufficiently amplified. Such PCR methods are described in e.g., U.S. Patent Nos. 4,965,188; 4,800,159; 4,683,202; 4,683,195. The product of the PCR is cloned and the clones containing the amplified DNA, derived by segregation of the primer extended strand, selected. Selection can be accomplished using the original primer as a hybridization probe.

Using the above methods, the sequence of the human gene immediately upstream of the translational start site of that portion of the gene, referred to herein as "exon III," was found to be homologous with rat and mouse cDNAs to a point at which a splice junction acceptor site was found. However, divergence of the human cDNA sequence upstream of the splice junction acceptor site indicated the presence of another exon(s), referred to herein as "exon I." The location of exon I was found by PCR to be about 5 kb upstream of the start of exon III. Using Southern blot analysis, an *Eco*RI fragment of about 9.4 kb was identified that contained both exon I and exon III. The 9.4 kb *Eco*RI fragment was cloned and propagated in the vector pBK-CMV, and excised therefrom and sequenced. Analysis of the nucleotide sequence of the fragment showed: (1) the presence of an exon I sequence greater than 90% homologous to the 5'-end of rat GDNF cDNA; (2) a 5' splice donor site at the 3'-end of the exon I sequence; (3) that exon I was about 5 kb upstream of the start of exon III; and (4) that the fragment had a region upstream of exon I that was approximately 79% identical to a limited region of the mouse GDNF gene (exon I to 140 bp upstream of exon I, GenBank entry #D88350S1). The GDNF promoter transcriptional start site was determined by 5'-end analysis of GDNF cDNAs.

The human proximal GDNF promoter DNA may be isolated in a manner similar to that described above. In particular, a cDNA originating from the proximal promoter can be identified and amplified from a human fetal kidney, brain or skeletal muscle polyA+ RNA by RT-PCR analysis using oligonucleotide primers based on the human GDNF encoding DNA as described in detail in Example 4.

Once produced, the DNA may then be incorporated into a vector for replication in a suitable host cell. The human distal GDNF promoter, the human proximal GDNF promoter and both the human distal and proximal GDNF promoters may be cloned into a suitable vector upstream from a reporter gene such as, for example, a luciferase gene. The GDNF promoter-luciferase constructs are each transfected into a human cell line which can subsequently be used to assay for promoter-regulated luciferase expression and to screen candidate compounds for human GDNF promoter-inducing activity as indicated by the production of luciferase. Of course, other modes of vector construction, as well as any of various reporters and cell lines, can be used to assess the promoter activity of the present invention.

In particular, vector construction employs methods known in the art. Generally, site-specific DNA cleavage is performed by treating with suitable restriction enzymes under conditions which generally are specified by the manufacturer of these commercially available enzymes. After incubation with the restriction enzyme, protein is denatured and extracted and the DNA recovered by precipitation. The cleaved fragments may be separated using, for example, polyacrylamide or agarose gel electrophoresis methods, according to methods known by those of skill in the art.

Sticky end cleavage fragments may be blunt ended using *E*. *coli* DNA polymerase 1 (Klenow) in the presence of the appropriate deoxynucleotide triphosphates (dNTPs) present in the mixture. Treatment with S1 nuclease also may be used, resulting in the hydrolysis of any single stranded DNA portions.

Ligations are performed using standard buffer and temperature conditions using T4 DNA ligase and ATP. Alternatively, restriction enzyme digestion of unwanted fragments can be used to prevent ligation. Ligation mixtures are transformed or transfected into a suitable host, and successful transfectants/transformants selected by drug resistance or other markers.

For example, standard vector constructs generally include reporter and/or selectable marker elements. Such elements are genes which confer a phenotype on a cell expressing the marker, such that the cell can be identified under appropriate conditions. Generally, reporter genes and selectable markers allow selection of transformed cells based on their ability to thrive in the presence or absence of a chemical or other agent that inhibits an essential cell function. Suitable markers, therefore, include genes coding for proteins which confer drug resistance or sensitivity thereto, impart color to, or change the antigenic characteristics of those cells expressing the selectable marker when the cells are grown in an appropriate selective medium. For example, selectable markers include: cytotoxic, antibiotic and drug resistance markers, whereby cells are selected by their ability to grow on media containing one or more of the cytotoxins, antibiotics or drugs; auxotrophic markers by which cells are selected by their ability to grow on defined media with or without particular nutrients or supplements; metabolic markers by which cells are selected for, e.g., their ability to grow on defined media containing the appropriate sugar as the sole carbon source, or markers which confer the ability of cells to form colored colonies on chromogenic substrates or cause cells to fluoresce.

Plasmids from the transfectants/transformants can then be prepared according to methods known to those in the art usually following a chloramphenicol amplification as reported by Clewell et al. (1972), *J*. *Bacteriol.* 110, 667. The DNA is isolated and analyzed usually by restriction enzyme analysis and/or sequencing. Sequencing may be by the well-known dideoxy method of Sanger et al. (1977), *Proc. Natl.* Acad. *Sci. USA* 74, 5463) as further described by Messing et al. (1981), *Nucleic Acid Res.* 9, 309, or by the method reported by Maxam et al. (1980), *Meth*. *Enzymol.* 65, 499.

Host cells are then transformed or transfected with the vectors of this invention. The vector may be in the form of a plasmid, a viral particle, a phage, etc. The host cells can be cultured in conventional nutrient media modified as appropriate for activating promoters, selecting transformants/transfectants or the like. The culture conditions, such as temperature, pH and the like, generally are similar to those previously used with the selected host cell and will be known to those of skill in the art.

Both prokaryotic and eukaryotic host cells may be used for cloning the desired oligonucleotide fragments. For example, among prokaryotic hosts, *Escherichia coli* is frequently used; however, other prokaryotic hosts such as strains of *Bacillus, Pseudomonas,* or the like, may be used if desired. Transfer vectors compatible with prokaryotic hosts can be derived from, for example, the plasmid pBR322 that contains operons conferring ampicillin and tetracycline resistance, and the various pUC vectors, that also contain sequences conferring antibiotic resistance markers. These markers may be used to obtain successful transformants by selection.

Eukaryotic hosts include yeast and mammalian cells in culture systems. *Pichia pastoris, Saccharomyces cerevisiae* and *S*. *carlsbergensis* are commonly used yeast hosts. Yeast-compatible vectors carry markers that permit selection of successful transformants by conferring protrophy to auxotrophic mutants or resistance to heavy metals on wild-type strains. Yeast compatible vectors may employ the 2-_ origin of replication (Broach et al. (1983), *Meth. Enzymol.* 101, 307), the combination of CEN3 and ARS1 or other means for assuring replication, such as sequences that will result in incorporation of an appropriate fragment into the host cell genome.

Mammalian cell lines available as hosts for cloning are known in the art and are available from depositories such as the American Type Culture Collection. These include but are not limited to human glioblastoma cells, neuroblastoma cells, HeLa cells, human embryonic kidney (HEK) cells, Chinese hamster ovary (CHO) cells, baby hamster kidney (BHK) cells, and others. Genes expressed in mammalian cells may also require transcription termination sequences and polyadenylation sequences, such as those derived from SV40, as described in Sambrook et al., *supra,* as well as a bovine growth hormone terminator sequence. Enhancer sequences which increase expression also may be included, and sequences which cause amplification of the reporter gene also may be desirable. These sequences are known in the art and include, for example, the mouse dihydrofolate reductase (*dhfr*) gene, placed adjacent to the coding sequence. Cells can then be selected for methotrexate resistance in dhfr-deficient cells. See, e.g. Urlaub et al. (1980), *Proc. Natl. Acad. Sci. USA* 77, 4216-4220; Ringold et al. (1981), *J. Mol. and Appl. Genet.* 1, 165-175. Vectors suitable for replication in mammalian cells may also include viral replicons, or sequences which ensure integration of the appropriate sequences comprising a GDNF promoter and a sequence encoding the reporter gene into the host genome.

Other eukaryotic systems are also known, as are methods for introducing polynucleotides into such systems, such as amphibian cells using methods well known in the art, insect cells using methods described in Summers and Smith, Texas Agricultural Experiment Station Bulletin No. 1555 (1987), and the like.

Transformation or transfection may be by any known method for introducing polynucleotides into a host cell, including packaging the polynucleotide in a virus and transducing a host cell with the virus, by direct uptake of the polynucleotide by the host cell, and the like, which methods are known to those skilled in the art. Such methods include DEAE dextran-mediated transfection, calcium phosphate precipitation, polylysine- or polyornithine-mediated transfection, or precipitation using other insoluble inorganic salts, such as strontium phosphate, aluminum silicates including bentonite and kaolin, chromic oxide, magnesium silicate, talc, and the like, electroporation, sonoporation, protoplast fusion, lipofection, peptoid delivery, or microinjection. See, e.g., Sambrook et al., *supra,* for a discussion of techniques for transforming and transfecting cells. The transformation or transfection procedures selected depend in part upon the host cell used and can be determined routinely by those of skill in the art.

Human GDNF promoter-reporter gene constructs expressed in or isolated from a recombinant host cell may be used to screen candidate compounds for their ability to stimulate reporter gene product expression and their potential to stimulate the expression *in vivo* of GDNF. One preferred method for identifying compounds that stimulate human GDNF promoter-controlled reporter gene expression comprises introducing into a cell a DNA construct that comprises a GDNF promoter operably linked to a reporter gene, mixing a test compound with the cell and measuring the level of expression of reporter gene product. A change in the level of expression of the reporter gene product indicates that the compound is capable of modulating the level of GDNF expression. The reporter gene construct is preferably stably integrated into the chromosomal DNA of the cell, but is also functional for the purposes disclosed herein in the form of an extrachromosomal element. The cell may be a eukaryotic cell, preferably a mammalian cell, more preferably a human cell, even more preferably a human cell that expresses GDNF, or any cell that contains the elements needed to express a structural gene under the regulatory influence of a mammalian gene promoter. In the examples provided herein, human glioblastoma U-87 MG and human neuroblastoma SK-N-AS cells were used as the hosts for promoter/reporter constructs because these cells produced GDNF, as determined by ELISA, and thus produced GDNF mRNA, as confirmed by RT-PCR.

Thus, for example, a test compound is evaluated for its ability to modulate luciferase expression, or for its ability to interfere with or augment the modulation of reporter gene expression by a human GDNF inducer compound, e.g., forskolin, or the like, in a- mammalian host cell that has been transfected with a human GDNF promoter-luciferase gene construct.

Although intact cells are preferred to evaluate candidate compounds for their ability to induce reporter gene expression by generation of an intracellular signal that results in the activation of the human GDNF promoter operably linked to the reporter gene, permeabilized cells may be used to test the efficacy of candidate compounds. Permeabilization of host cells that harbor the human GDNF promoter-reporter gene construct is effected by methods well known in the art.

Candidate compounds may also be evaluated for modulation of GDNF expression using the GDNF promoter-gene construct in an *in vitro* transcription assay as described by Sierra et al. "In vitro transcription with nuclear extracts from differentiated tissues," in Hames et al. (eds.) Gene Transcription: A Practical Approach (IRL Press, 1993), pp. 125-152.

Compounds that modulate GDNF expression by binding to the GDNF promoter or fragments thereof can be evaluated using techniques described in, for example, Gottesfeld et al. (1997), *Nature* 387, 202-205 and Heguy et al. (1995), *Gene Expression* 4,337-344.

Accordingly, agents found to stimulate the expression of the reporter gene product in cells transfected with a human GDNF promoter-reporter gene construct are considered potential therapeutic agents in several neurodegenerative disorders including, without limitation, Parkinson's disease, amyotrophic lateral sclerosis, epilepsy, Alzheimer's disease, or other conditions or diseases in which the absence or depressed level of naturally occurring GDNF may be an etiologic factor. In addition, such agents are potential therapeutic agents for supporting prenatal peripheral neuronal development as well as kidney ontogeny during embryonic development. Therapeutic agent efficacy is tested in any of a number of animal models of the above diseases, known in the art.

For example, the most extensively used animal models of Parkinson's disease replicate the neurodegeneration of dopaminergic neurons usually by administration of toxins. Unilateral injection of 6-hydroxydopamine (6-OHDA) into the substantia nigra of mice or rats results in neuronal loss in the ipsilateral striatum and substantia nigra pars compacta with little change in contralateral hemisphere. Similarly, methamphetamine-induced neurotoxicity results in neurodegeneration of dopaminergic and serotoninergic neurons and is considered by those of skill in the art to be closely aligned to the human condition. Efficacy of drugs is evaluated by behavioral outcome using the apomorphine-induced rotational behavior.

Another Parkinson's disease model is constructed using the neurotoxin N-methyl-4-phenyl- 1,2,3,6,-tetrahydropyridine (MPTP). MPTP has been administered to mice, rats, and monkeys. Administration of MPTP to monkeys results not only in loss of dopaminergic and serotoninergic neurons in substantia nigra pars compacta and striatum, but also in behavioral manifestations similar to those seen in human Parkinson's disease patients, such as akinesia and rigid posture.

In contrast to the above-described animal models of Parkinson's disease, a number of inbred strains of mice are available in which there is evidence of a gradual decline in dopaminergic cell numbers. For example, a D2 receptor-deficient mouse has been generated by homologous recombination whose behavioral characteristics resemble those of patients afflicted with Parkinson's disease. Fitzgerald et al. (1993), *Brain Res.* 608, 247-258. A second example is the weaver mutant mouse which shows a gradual decline in mesenchephalic dopaminergic neuron numbers over time up to 40% Verina et al. (1997), *Exp. Brain Res.* 113, 5-12; Adelbrecht et al. (1996), *Mol. Brain Res.* 43, 291-300; Mitsumoto et al. (1994), *Science* 265, 1107-1110.

Animal models of other neurodegenerative diseases have been described and are useful for evaluating the therapeutic efficacy of agents that are found to modulate the expression of the reporter gene in cells transfected with a human GDNF promoter-reporter gene construct as described herein. For example, Martin et al. (1995), *Brain Res.* 683, 172-178 describe an animal model of epilepsy, Matheson et al. (1997), *NeuroReport* 8, 1739-1742 and Oppenheim et al. (1995), *Nature* 373, 344-346 describe models of neurodegeneration that results from physical trauma, and Sagot et al. (1996), *J. Neurosci.* 16, 2335-2341 describe a model of motor neuron degeneration in animals.

Agents thus identified can be formulated into therapeutic compositions in a variety of dosage forms such as, but not limited to, liquid solutions or suspensions, tablets, pills, powders, ointments, suppositories, polymeric microcapsules or microvesicles, liposomes, and injectable or infusible solutions. The preferred form depends upon the mode of administration and the disease type targeted. The compositions also preferably include pharmaceutically acceptable vehicles, carriers or adjuvants, well known in the art, such as human serum albumin, ion exchangers, alumina, lecithin, buffer substances such as phosphates, glycine, sorbic acid, potassium sorbate, and salts or electrolytes such as protamine sulfate. Suitable vehicles are, for example, water, saline, dextrose, glycerol, ethanol, or the like, and combinations thereof. Actual methods of preparing such compositions are known, or will be apparent, to those skilled in the art. See, e.g., *Remington's Pharmaceutical Sciences,* Mack Publishing Company, Easton, Pennsylvania, 18th edition, 1990.

The above compositions can be administered using conventional modes of delivery including, but not limited to, intravenous, intramuscular, intraperitoneal, oral, intralymphatic, or subcutaneous administration.

Therapeutically effective doses will be easily determined by one of skill in the art and will depend on the severity and course of the disease, the patient's health and response to treatment, and the judgment of the treating physician.
In addition to the use of a human GDNF promoter DNA in a reporter gene construct to identify agents useful for treating neurodegenerative diseases such as Parkinson's disease, the human GDNF promoter can be used to design oligonucleotide probes to detect mutations that affect the expression of human GDNF for, e.g., diagnostic purposes. As used herein, the term "probe" refers to a structure comprised of a polynucleotide, as defined above, which contains a nucleic acid sequence complementary to a nucleic acid sequence present in a target polynucleotide. The polynucleotide regions of probes may be composed of DNA, and/or RNA, and/or synthetic nucleotide analogs. Such probes are useful in *in vitro* hybridization assays to distinguish a human GDNF wild-type promoter from a variant thereof and to screen for defects in the GDNF promoter, which may be diagnostic for Parkinson's disease, or other central and peripheral neurodegenerative diseases.

A probe will generally include about 6-8 to about 75 contiguous nucleic acids of the reference polynucleotide, generally about 10-12 to about 50 contiguous nucleic acids, and preferably about 15-20 to about 30 contiguous nucleic acids of the reference sequence. In particular, the probes for GDNF promoters are a length that allows the detection of unique sequences by hybridization.

Thus, using a determined portion of the isolated human distal GDNF promoter or of the isolated human proximal GDNF promoter, oligomers of approximately 6-8 or more nucleotides, which hybridize with the promoter, can be prepared using routine, standard methods such as excision or by, e.g., automated oligonucleotide synthetic methods. Such oligomers are useful, for example, for screening for the presence of a variant GDNF promoter in an individual who may be at risk for the development of Parkinson's disease, or as a prenatal screen for fetuses that may be similarly at risk for abnormal peripheral neuronal development or kidney ontogeny.

When the oligonucleotide probes are to be used as diagnostic reagents, the test sample to be analyzed, such as blood, serum or amniotic fluid may be treated such as to extract a nucleic acid fraction thereof. The resulting nucleic acid from the sample may be subjected to gel electrophoresis or other size separation techniques, or the nucleic acid sample may be dot-blotted without size separation. The sample is then exposed to an oligonucleotide probe that has been detectably labeled. Suitable labels and methods for attaching labels to probes are known in the art, and include but are not limited to radioactive labels incorporated by nick translation or kinasing, biotin, fluorescent and chemiluminescent probes, enzymes which catalyze the production of a detectable product such as horseradish peroxidase, alkaline phosphatase, β-galactosidase, and the like. The nucleic acids extracted from the sample are then treated with the labeled probe under conditions of suitable hybridization stringency.

The stringency of hybridization is determined by a number of factors during the washing procedure, including temperature, ionic strength, length of time and concentration of formamide. (Sambrook et al., *supra*.) Hybridization can be carried out by a number of techniques. Amplification of the sample nucleic acid, if required, can be performed, for example, by ligase chain reaction (LCR), polymerase chain reaction (PCR), Q-beta replicase, or other methods well known in the art. The amplified nucleic acids then may be detected using a hybridization assay, also well known in the art.

GDNF promoter polynucleotides or portions thereof can be provided in diagnostic kits. For example, oligomer probes capable of specifically hybridizing to a GDNF promoter polynucleotide can be packaged in diagnostic kits that include the probe nucleic acid sequence that may be labeled. Alternatively, the probe may be provided unlabeled and the ingredients for labeling could be included with the kit. The kit also may contain other suitably packaged reagents and materials needed or desirable for the particular hybridization protocol, for example, standards as well as instructions for performing the assay.

It is to be understood that while the invention has been described in conjunction with the preferred specific embodiments thereof, that the description above as well as the examples which follow are intended to illustrate and not limit the scope of the invention.

### III. Experimental

In the following examples, efforts have been made to ensure accuracy with respect to numbers used (e.g., amounts, temperature, etc.) but some experimental error and deviation should be accounted for. Temperature is always given in degrees C and, unless otherwise indicated, pressure is at or near atmospheric.

The following single-letter nucleotide abbreviations have been used throughout tc indicate the optional presence of either of a pair of nucleotides at a given location in a polynucleotide: R = G or A: Y = C or T; S = C or G; W = A or T; M = A or C; and K = G or T.

### Example 1

### Cloning of the Human Distal GDNF Promoter

### A. Demonstration of GDNF cDNA from human tissues.

Human GDNF cDNA was amplified by PCR from cDNA libraries from fetal brain, fetal kidney and adult skeletal muscle using a primer pair based on the rat GDNF cDNA sequence according to the method described in Schaar et al. (1994), *Exp. Neurol.* 130, 387-393. The sense and antisense primer pair were, respectively:
5'-GGT CTA CGG AGA CCG GAT CCG AGG TGC-3' (SEQ ID NO:3) and
5'-TCT CTG GAG CCA GGG TCA GAT ACA TC-3' (SEQ ID NO:4).
These results indicate that at least one human GDNF cDNA has a 5'-end substantially homologous to that of rat GDNF cDNA.

One such PCR-amplified cDNA from human fetal kidney (huGDNF) was cloned into pCR II (Invitrogen) and sequenced to give the sequence shown below. Nucleotide sequences 1-27 and 675-700 are homologous to the primer sequences. The sequence between nucleotides 50-674 is identical to GenBank entries L19062 and L19063.

### B. PCR walking of human genomic DNA.

DNA walking of human genomic DNA was performed with a PromoterFinder DNA walking kit (Clontech Laboratories, Palo Alto, Calif., USA), using the primers outGDNF1 (5'-CAG CAC CAG GCA GAC AGC-3' (SEQ ID NO:6)) and inGDNF1 (5'-GCC ACG ACA TCC CAT AAC TT-3' (SEQ ID NO:7)) as gene-specific primers 1 and 2, respectively. PCR was performed according to the manufacturer's instructions in the presence of 5% dimethyl sulfoxide. Amplified DNA was cloned into pCR II (Invitrogen, San Diego, Calif., USA) and sequenced. The nucleotide sequence of the amplified segment is shown below. Nucleotides 699-721 (in bold face) represent a translated sequence and nucleotides 702-721 are homologous to primer inGDNF1. Nucleotides 671-721 are highly homologous to those found in rat and mouse GDNF cDNAs (GenBank entries L15305 and U37372, respectively). The sequence diverges upstream from nucleotide 671. A potential splice junction acceptor site, shown under the sequence (YYYYYYYNY AGG) (SEQ ID NO:9), was found in the vicinity upstream of nucleotide 671 where the sequence diverges from rat and mouse cDNAs, indicating that another exon precedes this sequence.

### C. Cloning and identification of exon I of human GDNF and the GDNF promoter.

A human genomic library in the P1 bacteriophage vector pAd10*sac*BII (the Du Pont Merck Pharmaceutical Company human foreskin fibroblast P1 library no. 1; Sternberg (1992), *Trends in Genetics* 8, 11-16) was screened in pools by PCR using the primers GDNFg246F (5'-TAT GCC AGA GGA TTA TCC TGA TCA G-3' (SEQ ID NO:10)) and GDNFg246R (5'-TAG CCC AGA CCC AAG TCA GTG AC-3' (SEQ ID NO:11) as described by Schindelhauer et al. (1995), *Genomics* 28, 605-607) to identify human genomic clones containing exon IV of the human GDNF gene.

Clones DMPC-HFF#1-0575-F3, DMPC-HFF#1-0994-A6 and DMPC-HFF#1.-1332-H5 were identified and transformed into *E. coli* strain NS3516. Following isolation of DNA from these clones, the presence of exon III therein was confirmed by PCR using primer no. 5188 (5'-CCC TGC TTG AAA CTC TGA CC-3' (SEQ ID NO:12), nucleotides 400-419 of the sequence shown in Example 1B) and primer inGDNF1 (Example 1B, *supra*), as follows. Reaction mixtures contained 50 mM KCl, 10 mM Tris-HCl (pH 8.3 at 20°C), 1.5 mM MgCl₂, 0.2 mM dNTP, 1 µM of each primer, and 50 U/ml Taq DNA polymerase (Boehringer/Mannheim). Template DNA for amplification was provided by inoculating each 50 µl reaction mixture with bacteria from isolated colonies on Luria-Bertani (LB) agar plus 25 µg/ml kanamycin plates, where the bacteria contain the P1 clone of interest. Reaction mixtures were overlaid with mineral oil and amplified using the following scheme: initial denaturating step, 4 min at 94°C; 30 cycles of denaturing for 1 min at 94°C, annealing for 1 min at 60°C, and primer extension for 1 min at 72°C; final extension step, 10 min at 72°C. Each clone gave the expected about 322 bp product upon agarose gel electrophoresis. Bacteria with the vector alone gave no signal of this size, confirming the presence of exon III in each clone.

DNAs from genomic clones DMPC-HFF#1-0575-F3, DMPC-HFF#1-0994-A6 and DMPC-HFF#1-1332-H5, were individually digested with the restriction enzymes *Sma*I, *Nar*I, *Eco*RI*, Sal*I, *Bam*HI and *Bgl*II (each from Boehringer/Mannheim) in the presence of the buffer recommended by the supplier, and the consequent fragments resolved on a 0.5% agarose Tris/Borate/EDTA-buffered gel, and stained with ethidium bromide. The DNA in the gel was denatured, neutralized and transferred to a nylon membrane by capillary transfer overnight. After crosslinking the DNA to the nylon membrane using ultraviolet light and prehybridization for 16 hr at 42°C in a buffer having the composition 2% w/v blocking reagent (Boehringer/Mannheim, 5X SSC buffer (20X SCC buffer is 175 g NaCl and 88 g NaCitrate in 1 L water), 0.1% N-lauroylsarcosine, 0.02% sodium dodecyl sulfate (SDS) and 50% formamide as recommended by the supplier (Boehringer/Mannheim), the Southern blot was hybridized for 16 hr at 42°C with 10 ng/ml digoxigenin-labeled human GDNF cDNA as described in Example 1A, in a buffer similar to the prehybridization buffer. The membrane was washed twice for 15 min in 2X SSC containing 0.1% sodium dodecyl sulfate ("SDS") at room temperature, followed by a 30 min wash at 60°C in 0.1X SSC containing 0.1% SDS. Specific hybridization was detected with reagents and protocols provided by the supplier (Boehringer/Mannheim). *Eco*RI bands of about 9.4 kb, 2.7 kb and 0.7 kb were observed to hybridize with the human GDNF cDNA probe.

PCR primers were designed based on the GDNF sense sequence described by Schaar et al., *supra,* and the sequence upstream of exon III (see Example 1B) to map the position of the putative exon I relative to exon III. Primer no. 5231 (5'-GGT CTA CIG AGA CCG GAT CCG AGG T-3' (SEQ ID NO:13)) is a truncated form of the GDNF sense sequence designed to include a portion of a consensus splice junction donor site at its 3'-end, and containing a single 2'-deoxyinosine residue to destabilize a predicted stable hairpin loop that would otherwise have formed within the primer. Primer no. 5230 (5'-CGG CCC AAG CAA GGA CGG TGT TTC T-3' (SEQ ID NO:14)) is homologous to nucleotides 191-215 in the sequence shown in Example 1B about 480 bp upstream of the start of exon III. PCR amplification reaction mixtures contained 0.2 mM dNTPs, 0.6 _M each primers no. 5230 and 5231, 52.5 U/ml Expand DNA polymerase and a 1X concentration of the proprietary buffer provided by the supplier of the polymerase (Boehringer/Mannheim). Template DNA for amplification was either 200 ng human genomic DNA (Boehringer/Mannheim), 0.1 ng clone DMPC-HFF#1-0575-F3, or 0.1 ng of a *Sal*I deletion of this clone which removes about half of the insert DNA starting from a *Sal*I site about 1.75 kb downstream from the *Eco*RI site within exon IV of the human GDNF gene. Reaction mixtures of 50 µl each were overlaid with mineral oil and amplified using the following scheme: initial denaturing step, 3 min at 95°C; 20 cycles of denaturing for 1 min at 95°C, a 30-second ramp to 60°C, annealing for 1 min at 60°C, a 30-second ramp to 72°C, and primer extension for 4 min at 72°C; 15 cycles with similar times and temperatures except that the elongation time was increased incrementally by 15 seconds at each cycle; final extension step, 10 min at 72°C. Electrophoresis of an aliquot from each PCR reaction mixture showed approximately 4.5 kb products in each lane where template DNA was present, but no product where no template DNA was present. These results indicated that exon I is about 4.5 kb upstream from the position of hybridization of primer no. 5230 or about 5.0 kb upstream of the start of exon III.

The PCR amplification product from primers 5230/5231 was purified and about 100 ng of the product labeled with ^³²P-dCTP (Pharmacia Biotech oligolabelling kit). The Southern blot previously hybridized to digoxigenin-labeled human GDNF cDNA was hybridized overnight at 42°C with 1x10⁶ CPM/ml of the radiolabeled product in buffers similar to those previously described. Following washes as previously described, only the about 9.4 kb *Eco*RI fragment of each P1 genomic clone was detected by autoradiography. These results indicate that both exon I and exon III of the human GDNF gene reside within the same about 9.4 kb *Eco*RI fragment with the exons being separated by about 5.0 kb.

The about 9.4 kb *Eco*RI fragment was cloned into the vector pBK-CMV by initially cloning into *Eco*RI- and calf intestine alkaline phosphatase-treated Zap Express vector arms (Stratagene). Ten micrograms of P1 human genomic clone DMPC-HFF#1-0575-F3 was cleaved to completion with *Eco*RI. The fragments were resolved on a 0.5% agarose gel and purified from the gel. The *Eco*RI fragment was ligated into *Eco*RI- and calf intestine alkaline phosphatase-treated Zap Express vector arms. The ligation mixture was packaged into bacteriophage lambda particles with Gigapack II Gold packaging extracts (Stratagene). The packaged DNA was plated with XL-1 blue MRF' host cells in 0.7% NZY top agar onto NZY agar plates according to the supplier's instructions (Stratagene). Eleven isolated plaques were cored, the phage particles eluted overnight into 0.25 ml SM buffer and plasmid excised from lambda phage by coinfection of XL-1 blue MRF' cells with recombinant lambda phage and ExAssist f1 helper phage according to the suppliers instructions. Excised phagemids were rescued from filamentous phage particles by incubating XLOLR cells with the heat-treated excised pBK-CMV phagemid packaged as filamentous phage particles and plating on LB agar + 50 µg/ml kanamycin agar plates. After overnight incubation of the plates at 37°C, isolated colonies were picked and plasmid DNA Prepared from cultures grown in LB + 50 µg/ml kanamycin. One plasmid, pRBS*Eco*9.4#8 was characterized further.

### D. Sequencing the cloned 9.4 kb EcoRI fragment.

Plasmid pRBS*Eco*9.4#8 (see Figure 2) was sequenced initially utilizing primer no. 5231. Further sequencing was done using other primers that were synthesized based on the derived sequence. Initial sequencing revealed the sequence shown below. More extensive sequencing revealed the sequence shown in Figures 3A-3B (SEQ ID NO:1). This sequence was found to be 79% identical to a limited region of the mouse GDNF gene (exon I to 140 bp upstream of exon I, GenBank entry #D88350S1), using the BESTFIT program (Wisconsin Sequence Analysis Package, Genetics Computer Group, Madison, WI) with the default parameters suggested by the manufacturer.

### E. Determination of the transcriptional start site of the distal GDNF promoter by 5'-end analysis of GDNF cDNAs.

A CapFinder PCR cDNA synthesis kit (Clontech Laboratories) was used as the starting point in a modified RACE (rapid amplification of cDNA ends) procedure (Frohman (1989) *in* PCR Protocols (Academic Press, San Diego) pp. 28-38), to generate cDNA libraries enriched in GDNF cDNA 5'-ends. First strand cDNA synthesis on 0.5 µg of either human fetal kidney, fetal brain or adult skeletal muscle polyA+ RNA (Clontech Laboratories) was performed as described (CapFinder kit, Clontech Laboratories) except that cDNA synthesis was primed with 1 µl (50 ng/µl) random hexamers (Gibco/BRL). PCR was performed as described except that primers were 0.4 µM each of the supplied CapFinder PCR primer and GDNF anti-sense, with an initial denaturation at 94°C for 3 min. and cycled 24 times with denaturation for 1 min at 94°C annealing for 1 min. at 58°C, and primer extension for 1 min. at 72°C, with a final extension at 72°C for 10 min.

The primary PCR reactions were diluted 1000 fold into sterile distilled water and a 1 µl aliquot subjected to secondary amplification in similar reaction mixtures to those previously used, except in the presence of 0.2 µM CapFinder PCR primer and 0.4 µM primer no. 5191 (5'-GGT CAT CAT CAA AGG CGA TGG GT-3' (SEQ ID NO:16)) for 25 cycles of the same temperature program. A Southern blot of aliquots of the PCR reactions separated by agarose gel electrophoresis and probed with ³²P-ATP-labeled outGDNF1 (Example 1B, *supra*), showed multiple bands 600-1,100 bp in length. PCR products of this size range were purified by agarose gel electrophoresis, the DNA recovered and ligated into pCR 2.1 (Invitrogen). The ligation mixture was electroporated into ElectroMAX DH10B cells (Gibco/BRL) and plated onto LB agar + 50 mg/ml ampicillin + 50 mg/ml kanamycin plates. After overnight incubation at 37°C, colonies were lifted onto Nylon membranes, the DNA denatured, neutralized and uv cross-linked by standard methods. The filters were hybridized with a labeled cDNA probe as described in Example 1A, DNA prepared and sequenced from the hybridization positive colonies.

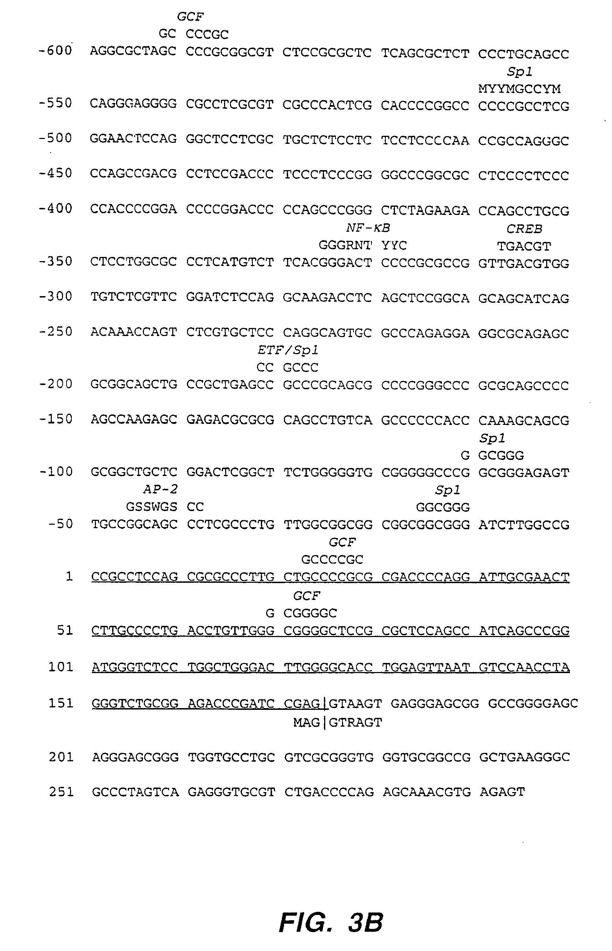

The sequence of a distal promoter-derived cDNA from human fetal kidney is shown below, in which nucleotides 1-174 correspond to exon I as shown in Figure 1 (nucleotides 1-174 in Fig. 3B), nucleotides 175-351 correspond to exon IIIb as shown in Figure 1, nucleotides 352-763 correspond to exon IV as shown in Figure 1, nucleotides 740-762 are contributed by primer #5191, and nucleotides 175-762 are identical with nucleotides 24-611 of the sequence given in Example 1A, *supra.*

### Example 2

### Preparation of a Human Distal GDNF Promoter-Reporter Gene Construct and Transfection of the Construct into E. coli.

In order to determine promoter activity, a reporter gene plasmid was prepared using the commercially available plasmid pGL3-Basic (Promega, Madison, WI, GenBank entry U47295) which harbors a firefly luciferase reporter gene, because of the sensitivity and linearity of the response to luciferase expressed therefrom. The distal GDNF promoter contained within plasmid pRBS*Eco*9.4#8 prepared as described in Example 1 was moved in a two-step method into the unique *Nhe*I restriction site of pGL3-Basic, upstream from the luciferase gene of the vector, as illustrated in Figure 5.

In the first step, plasmid pGL3-Basic was cleaved with restriction endonuclease *Nhe*I. The enzyme was inactivated by incubation at 65°C for 20 minutes, subsequently treated with shrimp alkaline phosphatase to prevent self-ligation of the vector, and this enzyme heat inactivated at 65°C for 15 minutes. Meanwhile, pRBS*Eco*9.4#8 was digested with *Avr*II followed by *Nhe*I. A 740-bp fragment was resolved by electrophoresis on a 0.5% agarose gel and the band recovered from the gel. The recovered *Avr*II/*Nhe*I fragment of the promoter was then ligated into the *Nhe*I cleaved pGL3-Basic. The ligated mixture was transformed into INVaF'-competent *E*. *coli* cells (Invitrogen) and plated onto LB ampicillin agar plates. After growth overnight at 37°C, plasmid DNA was prepared from individual colonies grown in small cultures of liquid LB ampicillin media. The orientation of the *Avr*II/*Nhe*I promoter fragment in pGL3-Basic was determined by digestion of plasmid DNA preparations with *Xba*I and by sequencing the junction sequence at the junction closest to the luciferase gene. The intermediate plasmid with the *Nhe*I/*Avr*II partial promoter fragment was called p *Nhe*-GDNF.

In the second step of the transfer of the distal GDNF promoter into pGL3-Basic, the intermediate plasmid p *Nhe*-GDNF was cleaved sequentially with the restriction enzymes *Nhe*I and *Pst*I, the mixture extracted with phenol/CHCl₃ and ethanol-precipitated. Plasmid pRBS*Eco*9.4#8 was cleaved with *Spe*I (which cleaves in the polylinker flanking the *Eco*RI insertion site for the genomic DNA insert) and *Pst*I. The about 1095 bp *Spe*I to *Pst*I fragment was resolved and purified by electrophoresis on a 0.5% agarose gel. The *Spe*I/*Pst*I promoter fragment was then ligated into p

*Nhe*-GDNF plasmid cleaved with *Nhe*I and *Pst*I. The fragment was transformed into INVaF'-competent *E*. *coli* cells (Invitrogen) which were plated onto LB + ampicillin plates and grown overnight at 37°C. Plasmid DNA was prepared from isolated colonies and the insertion of the promoter confirmed by restriction digestion and sequencing. The luciferase promoter plasmid containing the human GDNF promoter was called pGDNF-1635.

Controlled deletions of pGDNF-1635 are readily made for assessing the importance of various promoter or enhancer regions of the human distal GDNF promoter. Plasmid p *Nhe*-GDNF constitutes one such deletion of the 5'-terminal about 1,040 bp of the distal GDNF promoter from pGDNF-1635. A deletion of the 5'-terminal about 255 bp of the promoter is accomplished by digestion of pGDNF-1635 with the restriction endonuclease *Sac*I, inactivation of the enzyme and religation of the vector portion at low DNA concentrations, thus making p *Sac*-GDNF. Further deletions may be made by either manipulation with restriction enzymes or by controlled deletions generated with exonucleases as described by Henikoff (1984), *Gene* 28, 357. A map of the full-length human GDNF distal promoter and depictions of several 5' truncated promoter constructs are shown in Figure 7.

### Example 3

### Transfection of pGDNF-1635 into Human Cells and Assaying for Human Distal GDNF Promoter Activity

Human glioblastoma U-87 MG cells (ATCC number HTB-14) or neuroblastoma SK-N-AS cells (ATCC number CRL-2137) were transfected with the plasmid pGDNF-1635 prepared as described in Example 2 using the lipofection method (Felgner et al. (1987), *Proc. Natl. Acad. Sci. USA* 84, 7413-7417).

U-87 MG cells were grown to about 40% confluence in an 8.5 cm-diameter dish coated with rat tail collagen, type I (Collaborative Research, Cat. number 40450). The medium was removed, and the cells were washed once with phosphate-buffered saline (PBS), and gently overlayed with a solution containing 15 µg DNA plus 100 µg lipofectAMINE (Gibco/BRL number 18324-012) in 6.4 mls OptiMEM (Gibco/BRL number 31985) prepared according to the supplier's instructions. The cells were incubated for 5 hr, or up to 24 hr, at 37°C in a 5% CO₂ humidified atmosphere. The transfection solution was then removed and replaced with 10 mls of minimal essential medium (Gibco/BRL number 11095-080) containing 10% fetal bovine serum, 1% nonessential amino acids, 1% glutamine, 1% sodium pyruvate. After 48 hr incubation at 37°C, cells were collected, a cell extract prepared and assayed with luciferase assay reagents (Promega catalog number E1501).

SK-N-AS cells were grown to about 50% confluence in a 8.5-cm diameter dish, washed once with PBS and gently overlayed with a solution consisting of 10 µg DNA plus 80 µg lipofectAMINE (Gibco/BRL number 18324-012) in 6.4 mls OptiMEM (Gibco/BRL number 31985) prepared as described above. The cells were incubated at 37°C in a 5% CO₂ humidified atmosphere for 5 hr. The transfection solution was then removed and replaced with 10 mls Dulbecco's Modified Eagle's Medium (Gibco/BRL number 11965-092) containing 10% fetal bovine serum and 1% nonessential amino acids. After 48 hrs, cells were collected, a cell extract prepared and assayed with luciferase assay reagents (Promega catalog number E1501).

The effect of various promoter deletions on reporter gene expression were determined by minor modifications of the foregoing transfection procedures. When comparing expression of one promoter construct to another, it is important to account for variability of transfection efficiency between the two separate transfections. This has been accomplished here by co-transfection with another control plasmid expressing low amounts of a second, independently assayed reporter enzyme. After co-transfection with a mixture of the GDNF promoter deletion construct expressing firefly luciferase and the internal control reporter plasmid, both reporters are independently assayed and firefly luciferase activity is normalized by the activity of the control reporter enzyme. Variation in transfection efficiency between experiments is thereby eliminated. The control reporter plasmid utilized here was pRL-TK (Promega #E2241) which provides a low level of expression of a second independently assayable luciferase derived from the marine organism *Renilla*, under the control of the herpes simplex virus thymidine kinase promoter. Cell lysates are assayed sequentially for the two different luciferases under conditions of separate substrate addition and buffer composition, such that there is no spill-over between firefly and *Renilla* luciferase enzyme activities.

Human glioblastoma U-87 MG cells were transfected with a mixture of 13.5 micrograms of the reporter plasmids shown in Figure 7, and 1.5 micrograms pRL-TK internal control plasmid in the presence of 100 micrograms lipofectAMINE, as previously described for 24 hrs. Following transfection, cells were allowed to recover in the incubator for 20 hrs in U-87 MG medium (MEM containing 10% fetal bovine serum, 1% nonessential amino acids, 1% glutamine and 1% sodium pyruvate), before being removed from the 8.5 cm diameter plates and dispersed to rat tail collagen coated 12 well plates (Becton Dickinson Labware #40500) in the same medium. Plates were incubated for 9 hrs, at which time U-87 MG medium was removed and replaced with OptiMEM. Cells were incubated for 18 hrs at 37°C, media was removed, cells washed in chilled phosphate buffered saline, and a cell lysate prepared in situ by addition of 0.25 ml of 1X passive lysis buffer (Promega #E194A) to each well and incubation at room temperature for 15 minutes with gentle shaking of plates. SK-N-AS cells were transfected with the same plasmids as previously described.

Aliquots of 15 microliters of each cell lysate were assayed for firefly luciferase followed by *Renilla* luciferase, using reagents from a Dual-Luciferase reporter assay system kit (Promega #E1910). Luciferase activities were quantitated with the aid of a 96 well format luminometer (Dynex Technologies, model ML-3000) operating in the enhanced mode. Assays for each cell lysate were repeated in triplicate, while results for any given treatment were taken from the average of six repetitions of the treatment.

In order to positionally map possible enhancer and silencer elements as well as the basal promoter itself, 5' deletion mutants of pGDNF-1635 were constructed, transfected and luciferase activities determined. The results are shown in Figure 7. In U-87 MG glioblastoma cells, deletion of the most 5' 120 bp (-1516) resulted in a 30% decrease in promoter activity, indicating the presence of an enhancer element between -1635 and-1516. Further deletion to -1304 caused a 2-fold increase in promoter activity, suggestive of a silencer element between -1378 and -1304. Deletion of the next 170 bp to -1134, had no apparent effect in U-87 MG cells. Further deletion from -1134 to -366 resulted in incremental loss of promoter activity, indicating a broad region of enhancer elements. Interestingly, deletion within this region from -744 to -593, a region containing consensus binding sites for egr2 and AP-2, resulted in a more pronounced decrease in promoter activity, suggesting that egr2 and/or AP-2 are important to the regulatory activity. In contrast, deletion between -366 and -307, a region containing a consensus NF-κB binding site, resulted in no change in promoter activity in U-87 MG cells. Finally, deletion from -307 to -193 resulted in an about 5-fold increase in promoter activity, while further deletion to -62 resulted in slightly more activity, indicative of a strong silencer element(s) in this region.

Deletions of the regulatory region in SK-N-AS neuroblastoma cells produced notable differences as compared to the result obtained with U-87 MG cells. Figure 7 shows the presence of an enhancer in the first 120 bp, followed by a silencer at.-1378 to -1304 in SK-N-AS cells, similar to the results previously described for U-87 MG cells. However deletion of the region-1304 to -1134, which had no effect in U-87 MG cells, resulted in a decrease in SK-N-AS cells, suggesting the presence of a cell type-specific enhancer in this region; a pair of consensus binding sites for the transcription factor AP-2 were found in this region. Another difference between these two cell lines is found between -1134 and -852. Deletion of this region, previously shown to be an enhancer in U-87 MG cells, caused an increase in luciferase expression in SK-N-AS cells; a consensus binding site for CREB was found within this region. Proximal deletions between -852 and -593 revealed a region of enhancers in SK-N-AS cells similar in effect to that found in U-87 MG cells. Further deletion between -593 to -366 and -366 to -307 caused increased luciferase activity of 3 and 2 fold, respectively, in SK-N-AS cells, indicative of a silencer in this cell line. In contrast, U-87 MG cells showed that the region -593 to -366 appeared to function as an enhancer while -366 to -307, a region containing an apparent NF-κB binding site, had no effect. Taken together, the results suggest that cell type-specific elements may modify activity in this promoter.

### Example 4

### Cloning and Characterization of the Human Medial and Proximal GDNF Promoters

### A. Medial promoter-derived cDNA from human fetal kidney.

A CapFinder PCR cDNA synthesis kit (Clontech Laboratories) was used as the starting point in a modified RACE procedure to generate cDNA libraries enriched in GDNF cDNA 5'-ends. First-strand cDNA synthesis on 0.5 _g of either human fetal kidney, fetal brain or adult skeletal muscle polyA+ RNA (Clontech Laboratories) was performed according to the manufacturer's instructions (CapFinder kit, Clontech Laboratories) except that cDNA synthesis was primed with 1 µl (50 ng/µl) random hexamers (Gibco/BRL). PCR was performed according to instructions except that primers were the supplied CapFinder PCR primer and GDNF anti-sense, each present at 0.4 µM. The PCR program constituted an initial denaturation at 94°C for 3 min and 24 cycles of denaturation for 1 min at 94°C, annealing for 1 min at 58°C and primer extension for 1 min at 72°C, with a final extension at 72°C for 10 min.

The primary PCR reactions were diluted 1000-fold into sterile distilled water. A 1 µl aliquot of the each of the diluted reactions was subjected to secondary amplification in similar reaction mixtures to those previously used, except in the presence of 0.2 µM CapFinder PCR primer and 0.4 µM primer no. 5191 (5'-GGT CAT CAT CAA AGG CGA TGG GT-3' (SEQ ID NO:18)) for 25 cycles of the same temperature program. A Southern blot of aliquots of the secondary PCR reactions separated by agarose gel electrophoresis and probed with ^{.32}P-ATP-labeled outGDNF1 (Example 1B, *supra*) showed multiple bands 600-1,100 bp in length. PCR products of this size range were purified by agarose gel electrophoresis, the DNA recovered and ligated into plasmid pCR 2.1 (Invitrogen). The ligation mixture was electroporated into ElectroMAX DH10B cells (Gibco/BRL) and plated onto LB agar + 50 µg/ml ampicillin + 50 µg/ml kanamycin plates. After overnight incubation at 37°C, colonies were lifted onto Nylon membranes, the DNA denatured, neutralized and cross-linked to the membrane by exposure to UV light using standard methods. The filters were hybridized with a labeled cDNA probe (huGDNF from Example 1A, *supra*), and DNA was prepared and sequenced from the hybridization positive colonies. The sequence of one such cDNA derived from human fetal kidney is shown below. In the above sequence, nucleotides 585-607 are contributed by the primer no. 5191, nucleotides 1-97 (exon II, see Figure 1) and nucleotides 98-146 are homologous with nucleotides 5-101 and nucleotides 673-721 respectively, of the PCR walking fragment of human genomic DNA described in Example 1B and nucleotides 98-607 are homologous to nucleotides 24-611 in the cDNA sequence described in Example 1A, with a 78-bp deletion between nucleotides 122-201 thereof.

### B.Proximal promoter-derived cDNA from human fetal brain.

5' RACE was performed with a 5'-AmpliFINDER RACE kit (Clontech Laboratories) using random primed human fetal brain polyA+ RNA (Clontech Laboratories) and two rounds of PCR amplification utilizing nested sets of primers. The primary PCR amplification was performed with anchor primer (5'-CCT CTG AAG GTT CCA GAA TCG ATA G-3' (SEQ ID NO:20)) and gene-specific primer 1 (5'-GGT CAT CAT CAA AGG CGA TGG GT-3' (SEQ ID NO:16)) according to the supplier's suggested conditions, for 40 cycles using an annealing temperature of 54°C. Following 100-fold dilution into distilled water, 1 µl of the diluted primary PCR product was further PCR amplified for 30 cycles with primer no. 3442 (5'-GGA ACC TCG AGA ATC GAT AGT GAA TTC GTG-3' (SEQ ID NO:21)) and either gene-specific primer 2 (5'-TCG TAC GTT GTC TCA GCT GCA TC-3' (SEQ ID NO:22)) or gene-specific primer 3 (5'-ACC TTT TCC TCT GGA ATT CTC TG-3' (SEQ ID NO:23)). PCR conditions were similar to the primary PCR amplification except that an annealing temperature of 57°C was used. Amplified PCR products were cloned in the vector pCR2.1 (Invitrogen) and sequenced. A product from secondary amplification with primer no. 3442 and gene-specific primer 2 is shown below. In the above sequence, nucleotides 1-30 and 513-535 are contributed by primer no. 3442 and gene-specific primer 2, respectively, nucleotides 33-166 are homologous with nucleotides 588-721 of the PCR walking fragment of human genomic DNA described in Example 1B, and nucleotides 118-535 are homologous to nucleotides 24-519 in the cDNA sequence of Example 1A, with a 78-bp deletion between nucleotides 122-201.

### C. Sequence of the proximal promoter.

The sequence of the PCR walking fragment of human genomic DNA described in Example 1B was extended using as template the plasmid pRBSEco9.4#8, to obtain the promoter sequence of the proximal promoter shown in Figures 4A-4C (SEQ ID NO:2).

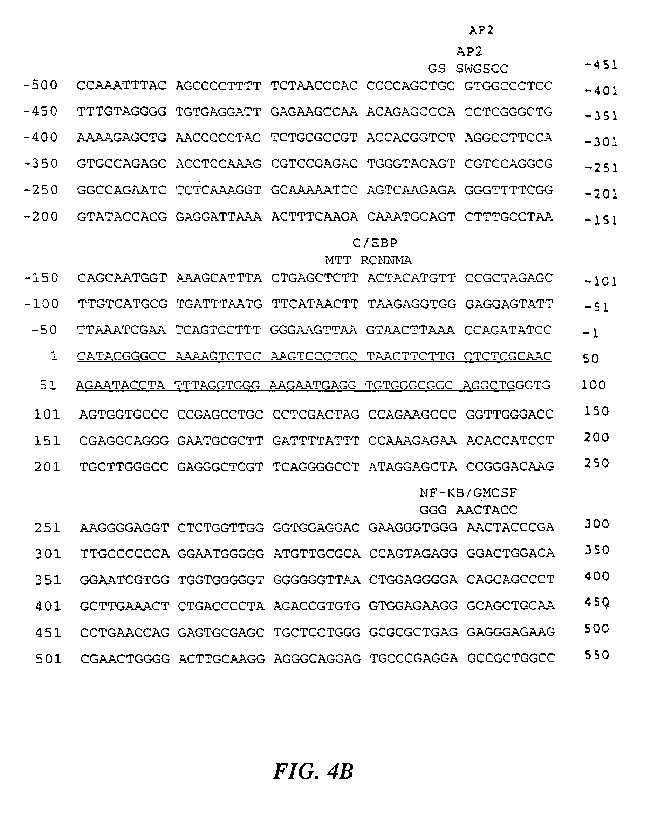

Positions +6 and +67 in the sequence shown in Figure 4B appear to be variable in different genomic DNAs; position +6 may be either A or G, and position +67 may be either T or C.

### Example 5

### Construction of a Luciferase Reporter Plasmid Containing Both Human Distal and Proximal GDNF Promoters

A reporter gene plasmid containing the human proximal and distal GDNF promoters and the gene encoding luciferase operably linked thereto was prepared according to the strategy depicted in Figures 6A-6B. The proximal and distal GDNF promoters contained within plasmid pRBSEco9. 4#8 were moved in two steps into the unique *Eco*RI and *Hind*III sites of the pGL3-Basic reporter plasmid derivative, pGDNF-1635. The procedure will first be briefly described, followed by a more detailed description.

First, the region between nucleotides -10 and +705 in the proximal promoter sequence shown in Figures 4A-4C, was modified by PCR to eliminate the translational initiation site at bp 696-698. The PCR product was then cloned into plasmid pKS bluescript II. The insert was removed from one clone, taking advantage of a neighboring *Hind*III site in the polylinker of the plasmid, as well as the unique *Eco*RV site within the insert. The *Hind*III to *Eco*RV insert fragment was then cloned into *Eco*RV- and *Hind*III-cleaved pRBS*Eco*9.4#8, to yield the plasmid pRBS*Eco*9.4proxmod. The insert from plasmid pRBS*Eco*9.4proxmod was removed by cleavage at the unique *Eco*RI and *Hind*III sites and cloned into *Eco*RI- and *Hind*III-cleaved pGDNF-1635, prepared as described in Example 2, to yield plasmid pGDNFprox+dist.

The translational initiation site in exon III was modified by PCR with the primer nos. 5361 (5'-CCA GAT ATC CCA TAC AGG CCA A-3' (SEQ ID NO:25)) and 5363 (5'-ATA ACT AGA TCT TAA AGT CCC GTC C-3' (SEQ ID NO:26)). PCR reactions contained 0.2 mM dNTPs, 0.4 _M each primer nos. 5361 and 5363, 70 U/ml Expand DNA polymerase and a 1X concentration of the proprietary buffer provided by the supplier of the polymerase (Boehringer/Mannheim). DNA template for amplification was 0.1 ng of plasmid pRB*SEco*9.4#8. Reactions of 50 µl each were overlaid with mineral oil, initially denatured at 94°C for 3 min and cycled 5 times with denaturation for 1 min at 94°C, annealing for 1 min at 42°C, and primer extension for 1 min at 72°C, followed by another 30 cycles with similar times and temperatures except that the annealing temperature was increased to 53°C, and finished with a final extension at 72°C for an additional 10 min. Electrophoresis of an aliquot from the PCR reaction showed the expected product of about 700 bp. Following cleanup of the PCR product (Promega MagicPrep), it was cloned into *Eco*RV-cleaved plasmid pBluescript II KS. The about 700-bp insert from one resulting plasmid, was released by digestion with *Eco*RV and *Hind*III, and the insert gel purified. This was cloned into pRB*SEco*9.4#8 which had been cleaved with *Eco*RV and *Hind*III. After selection on kanamycin-containing plates, and screening for the presence of an additional *Bgl*II site introduced into the plasmid via PCR, one resultant product plasmid, p*Eco*9.4proxmod, was selected for sequencing and further manipulation.

Plasmid p*Eco*9.4proxmod was cleaved with *Eco*RI and *Hind*III to release the insert of about 6.8 kb. This was ligated without gel purification of the fragment into *Eco*RI and *Hind*III cleaved pGDNF-1635 (this pGL3-Basic derivative was used because a unique *Eco*RI site, immediately inside the upstream polylinker segment, had been introduced during its construction). Transformants were selected on ampicillin-containing plates. One plasmid, pGDNFprox+dist with the desired insert, was used for further work.

### Example 6

### Construction of a Luciferase Reporter Plasmid Containing the Human Proximal GDNF Promoter

Elimination of the upstream portions of the promoters in pGDNFprox+dist resulted in reporter plasmids with the proximal (i.e., medial and proximal) promoters alone driving expression of luciferase. This was accomplished by cleavage of pGDNFprox+dist with restriction endonuclease *Mlu*I and re-ligation of the remaining plasmid, to yield pGDNFprox *Mlu*. Further deletions are made by cleavage of this plasmid (or pGDNFprox+dist) with *Mlu*I and another restriction enzyme which cleaves within the proximal promoter, but not in the remainder of the plasmid. For example, cleavage with *Mlu*I followed by cleavage with *Nru*I, conversion to blunt ends with Klenow fragment plus dNTPs, and subsequent re-ligation creates pGDNFprox *Nru.*

### Example 7

### Reverse Transcription-Polymerase Chain Reaction (RT-PCR) Results

RT-PCR was performed on various RNA preparations, using a common downstream primer and upstream primers specific for the distal, medial and proximal GDNF transcripts, in order to determine the relative abundance of the three transcripts in human tissues or U-87 MG cells. The distal primer, 5'-CCC AGG ATT GCG AAC TCT TGC-3' (SEQ ID NO:27) was identical to nucleotides 35 through 55 of the sequence shown in Example 1E. The medial primer, 5'-TCT CCA AGT CCC TGC TAA CTT CT-3' (SEQ ID NO:28) was identical to nucleotides 16 through 38 of the sequence shown in Example 4A. The proximal primer, 5'-CGT GCA TTC TGC GGT TCT-3' (SEQ ID NO:29) was identical to nucleotides 72 through 89 of the sequence shown in Example 4E. The common downstream primer, 5'-GAG CAG CAC CAG GCA GAC-3' (SEQ ID NO:30), was chosen in order to eliminate the complexity resulting from the alternative splicing which can occur at the exon II/exon III junction.

Total RNA was prepared from U-87 MG cells. U-87 MG cells were grown to confluence in 8.5 cm dishes as described in Example 3. The medium was then changed to OptiMEM (Gibco/BRL number 31985) supplemented with 0.5% bovine serum albumin, and the cells incubated for another 12 hrs in this medium, at which time either 10 ng/ml IL-1⁻, 10 nM phorbol didecanoate, 50 ng/ml basic FGF, 1 mM dibutyryl-cyclic AMP, 1 M dexamethasone, 10 ng/ml TNF^, or no additions (media) were made. The cells were incubated another 12 hrs, and total RNA was isolated using RNAzol^{™} B according to the manufacturer's instructions (Tel-Test, Inc., Friendswood, TX).

In order to eliminate possible contaminating genomic DNA in the RNA samples, they were first treated with DNase I. One microgram polyA⁺ mRNA from human fetal liver, adult liver, fetal kidney, fetal brain, adult brain or adult skeletal muscle (Clontech Laboratories) or 3 µg total RNA (U-87 MG cells), was pretreated with 1 unit DNase I (RQ1 RNase-free DNase, Promega #M610A) at 37°C for 30 minutes, followed by 20 minutes heat inactivation at 70°C, prior to first-strand cDNA synthesis. First-strand cDNA was synthesized from either 3 µg total RNA (U-87 MG cells) or 1 µg polyA⁺RNA (human tissue RNAs) with a Gibco SuperScript^{™} preamplification system kit (Gibco #18089-011) as directed, using 50 ng random primers. PCR was performed in 50 µl reaction volumes with 0.2 mM dNTPs, 1 µM distal, medial, proximal and common primers, 1 µl cDNA as template and 1 µl Advantage^{™} KlenTaq polymerase mix (Clontech #8417-1), in the presence of buffer supplied with the polymerase. Positive controls were provided by substitution of the 1 µl cDNA template with 1 µl containing either 0.1 attomole, 0.01 attomole or 0.001 attomole (1 zeptomole = 1 x 10⁻²¹ mole) of one of the distal, medial or proximal cDNAs (Examples 1E, 4A and 4E, respectively) or with equimolar amounts of each of the above cDNAs. Negative controls consisted of substitution of the 1 µl cDNA template with 1 µl of a similar reaction where the addition of reverse transcriptase was omitted. Temperature cycling parameters consisted of initial denaturation at 94°C for 2 minutes, followed by 30 cycles of denaturation at 94°C for 30 seconds, annealing at 66°C for 30 seconds, and extension at 72°C for 1 minute, followed by a final incubation at 72°C for 10 minutes. Ten microliter aliquots of the PCR reactions were applied to 4% NuSieve® 3:1 Agarose gels in TBE buffer (FMC BioProducts #54906) and resolved by electrophoresis. The gels were visualized and photographed with the aid of a transilluminator at 302 nm.

Figure 8A shows the validation results of the RT-PCR. Lane 1. shows a band of a size that is in agreement with the predicted 114 bp which is the result of amplification of 0.1 attomole of the cloned proximal transcript from Example 4E with a mixture of the primers. Lane 2 shows a band which is in agreement with the predicted 150 bp product, resulting from amplification of 0.1 attomole of the cloned medial transcript from Example 4A with the primer mixture. Lane 3 shows a band which is in agreement with the predicted 208 bp product, resulting from amplification of 0.1 attomole of the cloned distal transcript from Example 1E with the primer mixture. The product bands in lanes 1, 2 and 3 are of approximately equal intensity suggesting that amplification of each product takes place with similar efficiency under these conditions. Lane 4 shows that no product is found when no template is added. Lane 5 is the result of amplification of a mixture of 0.1 attomole each of the proximal, medial and distal templates, with the primer mixture. Lanes 6 and 7 are similar to lane 5 except that each template is present at 0.01 attomole (lane 6) or 0.001 attomole (1 zeptomole, lane 7). Lanes 5, 6 and 7, taken together, show that amplification of each product takes place with similar efficiency and that the sensitivity of the assay is 1 zeptomole or less, for any of the three templates. Separate experiments where 0.1 attomole of proximal template was cycled in the presence of medial, distal and common primers only, or 0.1 attomole of medial template was cycled in the presence of proximal, distal and common primers only, or 0.1 attomole of distal template was cycled in the presence of proximal, medial, and common primers only, showed no products, indicating an absence of cross-reactivity between dissimilar templates and primers (results not shown).

Figure 8B shows the results of RT-PCR with the proximal, medial and distal transcript primers on RNA isolated from U-87 MG cells after various treatments. Lane 1 contains the products after amplification of a mixture of 0.1 attomole of each template. Lane 4 shows the RT-PCR products from amplification of RNA isolated from U-87 MG cells in the presence of media alone, while lane 3 is a similar reaction except that reverse transcriptase was not added to the cDNA synthesis reaction (-RT control). Approximately equal amounts of products from the distal and proximal amplification are found in lane 4, whereas lane 3 shows no products indicating that formation of products is dependent on the presence of reverse transcriptase. Lanes 5, 6, 7 and 8, show that 12 hrs treatment of U-87 MG cells with 10 ng/ml interleukin-1- (IL-1⁻), 10 nM phorbol didecanoate (PDD), 50 ng/ml basic fibroblast growth factor (bFGF) or 1 mM dibutyryl-cAMP (db-cAMP), caused increases relative to vehicle alone (lane 4) in the amount of proximal and distal transcripts of GDNF. In lanes 5-8, the proportion of proximal and distal transcripts is approximately equivalent with slightly more distal than proximal products evident in each lane. Lane 9 shows that 12 hrs treatment of U-87 MG cells with 1 M dexamethasone decreases the amount of proximal and distal transcripts of GDNF relative to vehicle alone. Finally, lane 10 shows that 12 hrs treatment of U-87 MG cells with 10 ng/ml tumor necrosis factor ^ (TNF^) increases the amount of proximal and distal transcripts of GDNF relative to vehicle alone. Thus, both proximal and distal transcripts of GDNF, but not the medial transcript are evident in U-87 MG cells and the amounts of these transcripts can be increased by .treatment with IL-1⁻, PDD, bFGF, db-cAMP or TNF^, while dexamethasone decreases the transcripts.

Figure 8C shows the result of RT-PCR with the proximal, medial and distal primer mixture, on RNA isolated from various human tissues. Lanes 3 through 8, containing the products from amplification of RNA from fetal liver, adult liver, fetal kidney, adult brain, fetal brain and adult skeletal muscle, respectively, show only the presence of the distal transcript derived product. Fetal kidney (lane 5) and adult skeletal muscle (lane 8), show approximately equal and the highest concentration of product, while fetal brain (lane 7), fetal liver (lane 3), and adult liver (lane 4), show progressively less product. Adult brain (lane 6) shows a just detectable product. Control reactions where reverse transcriptase was omitted from the cDNA synthesis reaction, showed no product for all of the above tissues (results not shown). Thus, in contrast to U-87 MG cells, these tissues contain only the distal transcript of GDNF.

The RT-PCR results shown in Figure 8A show that this assay is both sensitive and specific for three transcripts of GDNF. The results from Figure 8B indicate that both proximal and distal transcripts in approximately equal proportions, are readily detected in U-87 MG cells treated in various ways. However, RT-PCR of RNA isolated from selected human tissues detected nearly exclusively the presence of the distal transcript, due to distal promoter activity. It is possible that other transcripts are present in these human tissues, but the distal transcript is present in far higher concentrations, such that it is the only one detected under these conditions of amplification. Furthermore, the U-87 MG cells may represent a specific cell type or a developmental stage which is not present in this limited sampling of tissues. The results presented here suggest that the distal promoter is a major determinant of GDNF expression *in vivo.*

Thus, distal and proximal human GDNF promoter and reporter gene constructs comprising one, two or three promoters have been disclosed. Although preferred embodiments of the subject invention have been described in some detail, it is to be understood that obvious variations can be made without departing from the spirit and the scope of the invention as defined by the appended claims.

A deposit of biologically pure cultures of *E*. *coli* transformant XLOLR/pRBS*Eco*9.4#8 obtained in Example 1C was made with the American Type Culture Collection (ATCC), 12301 Parklawn Drive, Rockville, Maryland, USA, on July 15, 1997, and assigned Accession Number 98498. The deposit was made under the provisions of the Budapest Treaty. Should there be a discrepancy between the sequence presented in the present application and the sequence of the gene of interest in the deposited plasmid due to routine sequencing errors, the sequence in the deposited plasmid controls.

## Claims

1. A DNA having human distal glial cell line-derived neurotrophic factor (GDNF) promoter function and comprising the nucleotide sequence set forth at positions -62 through -1, or -363 through -1, or -1635 through -1 of Figures 3A-3B (corresponding to nucleotides 1575 to 1636, 1274 to 1636, or 1 to 1636, respectively of SEQ ID NO:1).

2. The DNA as claimed in claim 1 operably linked to a reporter gene.

3. The DNA as claimed in claim 2, wherein the reporter gene encodes a polypeptide selected from the group consisting of chloramphenicol acetyl transferase, β-galactosidase, luciferase, alkaline phosphatase, human growth hormone, and green fluorescent protein.

4. A vector comprising a DNA as claimed in any one of claims 1 to 3.

5. A host cell comprising a vector as claimed in claim 4.

6. The host cell of claim 5, wherein the host cell is a mammalian cell.

7. The host cell of claim 6, wherein the host cell is able to expresses GDNF.

8. The host cell of any one of claims 5 to 7, wherein the DNA as claimed in any one of claims 1 to 3 is integrated into the chromosomal DNA of the host cell.

9. A method for identifying a compound that is capable of modulating the expression of a human distal glial cell line-derived neurotrophic factor (GDNF), comprising:
(a) introducing into a host cell a DNA as claimed in claim 2 or claim 3;
(b) mixing a test compound with the host cell and culturing said cell under conditions whereby the reporter gene is capable of expression; and
(c) comparing the level of expressed reporter gene product in the presence and absence of the test compound;
wherein a change in the level of expression of the reporter gene product indicates that the test compound is capable of modulating the level of GDNF expression.

10. The method of claim 9, wherein the host cell is a mammalian cell that expresses GDNF.

11. The method of claim 10, wherein the mammalian cell is selected from the group consisting of a U-87 MG cell and an SK-N-AS cell.

12. A diagnostic test kit comprising an oligonucleotide capable of specifically hybridizing to a DNA as claimed in any one of claims 1 to 3.

13. The use of a DNA as claimed in claims 2 or 3 for identifying a compound that is capable of modulating the expression of a human glial cell line-derived neurotrophic factor (GDNF).

## Patentansprüche

1. DNA, die eine Funktion als distaler Promoter des menschlichen Gliazellen-abgeleiteten neurotrophen Faktors (GDNF) aufweist und die Nucleotidsequenz, die in den Positionen -62 bis -1 oder -363 bis -1 oder -1635 bis -1 der Figuren 3A bis 3B (entsprechend jeweils den Nucleotiden 1575 bis 1636, 1274 bis 1636 oder 1 bis 1636 der SEQ-ID-Nr.: 1) dargestellt ist, umfaßt.

2. DNA nach Anspruch 1, die funktionell mit einem Reportergen gekoppelt ist.

3. DNA nach Anspruch 2, worin das Reportergen ein Polypeptid codiert, ausgewählt aus der Gruppe, bestehend aus Chloramphenikolacetyltransferase, β-Galactosidase, Luciferase, alkalischer Phosphatase, menschlichem Wachstumshormon und grünem fluoreszierenden Protein.

4. Vektor, umfassend eine DNA nach einem der Ansprüche 1 bis 3.

5. Wirtszelle, umfassend einen Vektor nach Anspruch 4.

6. Wirtszelle nach Anspruch 5, wobei die Wirtszelle eine Säugerzelle ist.

7. Wirtszelle nach Anspruch 6, wobei die Wirtszelle GDNF exprimieren kann.

8. Wirtszelle nach einem der Ansprüche 5 bis 7, wobei die DNA nach einem der Ansprüche 1 bis 3 in die chromosomale DNA der Wirtszelle integriert ist.

9. Verfahren zur Identifikation einer Verbindung, die die Expression eines menschlichen distalen Gliazellen-abgeleiteten neurotrophen Faktors (GDNF) modulieren kann, umfassend:
(a) Einführen von DNA nach Anspruch 2 oder Anspruch 3 in eine Wirtszelle;
(b) Mischen der Testverbindung mit der Wirtszelle und Kultivieren der Zellen unter Bedingungen, bei denen das Reportergen exprimiert werden kann;
(c) Vergleichen des Niveaus des in Gegenwart und in Abwesenheit der Testverbindung exprimierten Reportergenproduktes;
wobei die Veränderung des Expressionsniveaus des Reportergenproduktes anzeigt, daß die Testverbindung den GDNF-Expressionsgrad modulieren kann.

10. Verfahren nach Anspruch 9, wobei die Wirtszelle eine Säugerzelle ist, die GDNF exprimiert.

11. Verfahren nach Anspruch 10, wobei die Säugerzelle aus der Gruppe, bestehend aus einer U-87 MG-Zelle und einer SK-N-AS-Zelle, ausgewählt ist.

12. Diagnostitsches Testkit, umfassend ein Oligonucleotid, das DNA nach einem der Ansprüche 1 bis 3 spezifisch hybridisieren kann.

13. Verwendung von DNA nach den Ansprüchen 2 oder 3 zur Identifizierung einer Verbindung, die die Expression eines menschlichen Gliazellen-abgeleiteten neurotrophen Faktors (GDNF) modulieren kann.

## Revendications

1. ADN ayant une fonction de promoteur du facteur neurotrophique dérivé d'une lignée cellulaire gliale distale humaine (GDNF) et comprenant la séquence nucléotide définie aux positions -62 jusqu'à -1, ou -363 jusqu'à -1, ou -1635 jusqu'à -1 des Figures 3A-3B (correspondant aux nucléotides 1575 à 1636, 1274 à 1636 ou 1 à 1636, respectivement, de SEQ ID n° 1.

2. ADN selon la revendication 1, lié de façon opérante à un gène rapporteur.

3. ADN selon la revendication 2, dans lequel le gène rapporteur code pour un polypeptide choisi dans le groupe consistant en chloramphénicol acétyl transférase, β-galactosidase, luciférase, phosphatase alcaline, hormone de croissance humaine et protéine fluorescente verte.

4. Vecteur comprenant un ADN selon l'une quelconque des revendications 1 à 3.

5. Cellule hôte comprenant un vecteur selon la revendication 4.

6. Cellule hôte de la revendication 5, dans laquelle la cellule hôte est une cellule de mammifère.

7. Cellule hôte de la revendication 6, dans laquelle la cellule hôte est capable d'exprimer le GDNF.

8. Cellule hôte selon l'une quelconque des revendications 5 à 7, dans laquelle l'ADN selon l'une quelconque des revendications 1 à 3 est intégré dans l'ADN chromosomique de la cellule hôte.

9. Procédé pour identifier un composé qui est capable de moduler l'expression d'un facteur neurotrophique dérivé d'une lignée cellulaire gliale distale humain (GDNF), comprenant :
(a) l'introduction dans une cellule hôte d'un ADN selon la revendication 2 ou la revendication 3 ;
(b) le mélange d'un composé test avec la cellule hôte et la mise en culture de ladite cellule dans les conditions dans lesquelles le gène rapporteur est capable d'expression ; et
(c) la comparaison du niveau du produit du gène rapporteur exprimé en présence et en l'absence du composé test ;
dans lequel une modification dans le niveau d'expression du produit du gène rapporteur indique que le composé test est capable de moduler le niveau de l'expression GDNF.

10. Procédé selon la revendication 9, dans lequel la cellule hôte est une cellule de mammifère qui exprime GDNF.

11. Procédé selon la revendication 10, dans lequel la cellule de mammifère est choisie dans le groupe consistant en une cellule U-87 MG et une cellule SK-N-AS.

12. Kit de test diagnostic comprenant un oligonucléotide capable de s'hybrider spécifiquement à un ADN selon l'une quelconque des revendications 1 à 3.

13. Utilisation d'un ADN selon les revendications 2 ou 3 pour identifier un composé qui est capable de moduler l'expression d'un facteur neurotrophique dérivé-souche cellulaire gliale humain (GDNF).
